(19) Europäisches Patentamt
European Patent Office
Office européen des brevets

(11) EP 4 293 015 A1

(12) EUROPEAN PATENT APPLICATION
published in accordance with Art. 153(4) EPC

(43) Date of publication:
**20.12.2023 Bulletin 2023/51**

(21) Application number: **22758865.4**

(22) Date of filing: **22.02.2022**

(51) International Patent Classification (IPC):
***C07D 235/22*** $^{(2006.01)}$ ***C07D 235/04*** $^{(2006.01)}$
***C07D 235/02*** $^{(2006.01)}$ ***C07D 235/00*** $^{(2006.01)}$
***A01N 43/52*** $^{(2006.01)}$ ***A01P 7/00*** $^{(2006.01)}$

(52) Cooperative Patent Classification (CPC):
**A01N 43/52; A01P 7/00; A01P 7/02; A01P 7/04; C07D 235/00; C07D 235/02; C07D 235/04; C07D 235/22**

(86) International application number:
**PCT/CN2022/077277**

(87) International publication number:
**WO 2022/179501 (01.09.2022 Gazette 2022/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.02.2021 CN 202110204832**

(71) Applicant: **Qingdao Kangqiao Pesticides and Chemicals Group Co., Ltd**
**Shandong 266011 (CN)**

(72) Inventors:
- **ZHANG, Laijun**
  **Qingdao Shandong 266011 (CN)**
- **LIU, Yu**
  **Qingdao Shandong 266011 (CN)**
- **LIU, Ranjin**
  **Qingdao Shandong 266011 (CN)**
- **CUI, Yingrui**
  **Binzhou, Shandong 256500 (CN)**
- **WANG, Yuqi**
  **Qingdao Shandong 266011 (CN)**
- **WANG, Shiling**
  **Binzhou, Shandong 256500 (CN)**
- **FENG, Ruijie**
  **Binzhou, Shandong 256500 (CN)**
- **ZHAO, Zhanru**
  **Qingdao Shandong 266011 (CN)**
- **WEI, Xinhao**
  **Qingdao Shandong 266011 (CN)**
- **LI, Yahui**
  **Qingdao Shandong 266011 (CN)**
- **DU, Yaoyao**
  **Qingdao Shandong 266011 (CN)**
- **GONG, Chen**
  **Qingdao Shandong 266011 (CN)**
- **SUN, Yunxiao**
  **Binzhou, Shandong 256500 (CN)**
- **FAN, Shien**
  **Binzhou, Shandong 256500 (CN)**
- **GUO, Qiangqiang**
  **Qingdao Shandong 266011 (CN)**
- **GUO, Wei**
  **Qingdao Shandong 266011 (CN)**
- **GAO, Jie**
  **Binzhou, Shandong 256500 (CN)**
- **LIU, Yingshuai**
  **Binzhou, Shandong 256500 (CN)**
- **LI, Ning**
  **Binzhou, Shandong 256500 (CN)**

(74) Representative: **Gerauer, Marc Philippé**
**Kraus & Weisert**
**Patentanwälte PartGmbB**
**Thomas-Wimmer-Ring 15**
**80539 München (DE)**

# (54) BENZIMIDAZOLE COMPOUND OR SALT THEREOF, PREPARATION METHOD THEREFOR AND USE THEREOF, AND INSECTICIDE AND ACARICIDE AND USE THEREOF

(57) Provided are a benzimidazole compound as represented by fonnula (I) or a salt thereof, a preparation method therefor, and the use thereof. The benzimidazole compound or the salt thereof has excellent insecticidal and acaricidal effects, and can display acaricidal effects especially when same is used at a low concentration.

EP 4 293 015 A1

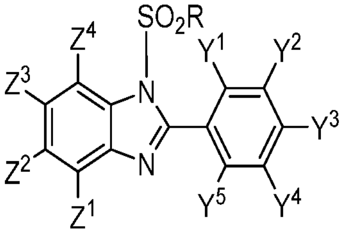

(I)

## Description

### Cross Reference to Related Applications

This application claims the benefit of Chinese patent application 202110204832.5, filed on February 23, 2021, the contents of which are incorporated herein by reference.

### FIELD

The present disclosure relates to the field of insecticides, in particular to a benzimidazole compound or a salt thereof, a preparation method therefor and use thereof, and an insecticide and acaricide and use thereof.

### BACKGROUND

In crop production in agriculture and horticulture and the like, losses due to pests and the like are still great, pests having resistance to existing drugs occur, and from the viewpoints of influence on environmental organisms and saving labor and the like, it is expected to develop an insecticide and acaricide for agricultural horticulture having new effects, less influence on natural enemies and beneficial insects, osmotic transfer activity and the like.

Advanced Materials Research Vols 236-238 (2011) pp2570-2573 reports the following benzimidazoles, and although some compounds have anti-inflammatory activity, they are not disclosed as relating to insecticidal and acaricidal activity.

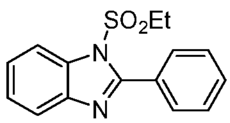
$KC_1$

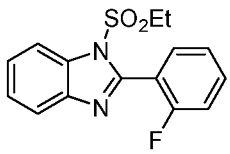
$KC_2$

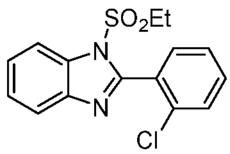
$KC_3$

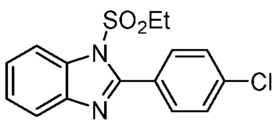
$KC_4$

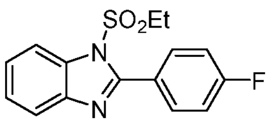
$KC_5$

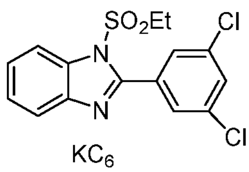
$KC_6$

Currently, there is a need to provide a compound having a highly effective insecticidal and acaricidal effect when used at a low concentration.

### SUMMARY

An object of the present disclosure is to overcome the defects that the existing benzimidazole compound can have insecticidal and acaricidal activity only when used at a high concentration, and it is difficult to meet the control requirements of pest mites in a field, and to provide a novel benzimidazole compound and a salt thereof, which are expected to be used as an active ingredient in an insecticide and acaricide, and can have a highly effective controlling effect when used at a low concentration (not higher than 100 ppm).

To achieve the above object, a first aspect of the present disclosure provides a benzimidazole compound or a salt thereof, wherein the benzimidazole compound has a structure represented by a formula (I):

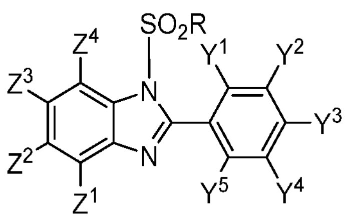
Formula (I);

wherein in the formula (I),

R is selected from substituted or unsubstituted $C_1$-$C_{10}$ alkyl, substituted or unsubstituted $C_3$-$C_{10}$ cycloalkyl, substituted or unsubstituted $C_2$-$C_{10}$ alkenyl, and substituted or unsubstituted $C_2$-$C_{10}$ alkynyl, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy;

$Y^1$, $Y^2$, $Y^3$, $Y^4$, and $Y^5$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3- to 8-membered ring;

$Z^1$, $Z^2$, $Z^3$, and $Z^4$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

A second aspect of the present disclosure provides a method for preparing a benzimidazole compound, including:

(1) subjecting a compound V and a compound IV to a first reaction in a first solvent in the presence of a first alkaline substance and a condensing agent to obtain a compound III;

(2) subjecting the compound III and an acidic substance to a second reaction in a second solvent to obtain a compound II; and

(3) subjecting the compound II and a sulfonyl-containing compound to a third reaction in a third solvent in the presence of a second alkaline substance to obtain the benzimidazole compound; wherein the compound V has a structure represented by a formula (V), the compound IV has a structure represented by a formula (IV), the compound III has a structure represented by a formula (III), the compound II has a structure represented by a formula (II), the

benzimidazole compound has a structure represented by a formula (I), and the sulfonyl-containing compound has a structure represented by a formula (VI);

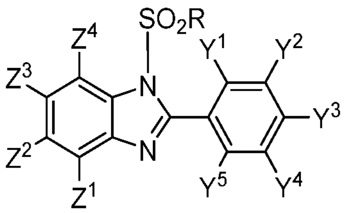
Formula (I);
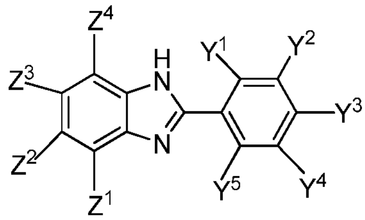
Formula (II);

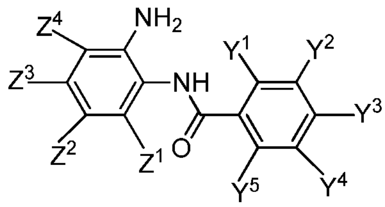
Formula (III);
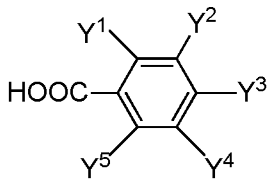
Formula (IV);

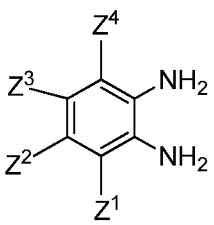
Formula (V);
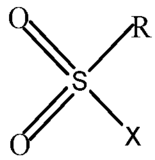
Formula (VI);

in the formula (I), the formula (II), the formula (III), the formula (IV), the formula (V) and the formula (VI), definitions of R, $Y^1$, $Y^2$, $Y^3$, $Y^4$, $Y^5$, $Z^1$, $Z^2$, $Z^3$, and $Z^4$ are correspondingly the same as those in the first aspect, and X is selected from halogen.

A third aspect of the present disclosure provides a benzimidazole compound prepared by the method described in the second aspect.

A fourth aspect of the present disclosure provides a use of the benzimidazole compound or the salt thereof according to the first aspect or the third aspect of an insecticide and acaricide.

A fifth aspect of the present disclosure provides an insecticide and acaricide, including an active ingredient selected from at least one of the benzimidazole compound or the salt thereof according to the first aspect or the third aspect.

A sixth aspect of the present disclosure provides use of the insecticide and acaricide for killing pests and/or mites in agriculture, forestry and horticulture.

Compared with the prior art, the present disclosure has at least the following advantages:
the benzimidazole compound or the salt thereof provided by the present disclosure is used as an insecticide and acaricide as an active ingredient, and can have excellent control effects when used at a low concentration.

Other features and advantages of the present disclosure will be described in detail in the subsequent specific embodiments.

## DETAILED DESCRIPTION

The endpoints and any values of the ranges disclosed herein are not limited to the precise range or value, and these ranges or values should be understood as including values close to these ranges or values. For numerical ranges, the endpoint values of each range, the endpoint values of each range and individual point values, and individual point values may be combined with each other to obtain one or more new numerical ranges, and these numerical ranges should be considered to be specifically disclosed herein.

In the present disclosure, the terms involved are collectively explained as follows: halogen refers to fluorine, chlorine, bromine, and iodine.

Alkyl refers to a linear or branched alkyl group, excluding cycloalkyl, and the $C_1$-$C_8$ alkyl refers to an alkyl group having 1 to 8 carbon atoms, and includes, for example, but is not limited to, methyl, ethyl, n-propyl, isopropyl, n-butyl, isobutyl, sec-butyl, tert-butyl, n-pentyl, isopentyl, n-hexyl, and the like. Cycloalkyl refers to alkyl containing a cyclic chain, and the $C_1$-$C_8$ cycloalkyl refers to a cycloalkyl group having 1 to 8 carbon atoms, and includes, for example,but is not limited to, cyclopropyl, cyclobutyl, cyclopentyl, cyclohexyl and the like.

Alkenyl refers to linear or branched alkenyl, and the $C_2$-$C_8$ alkenyl refers to an alkenyl group having 2 to 8 carbon atoms, and includes, for example, but is not limited to, 1-propenyl, 2-propenyl and different butenyl, pentenyl and hexenyl isomers; and alkenyl also includes polyene such as 1,2-propadienyl and 2,4-hexadienyl.

Alkynyl refers to linear or branched alkynyl, and the $C_2$-$C_8$ alkynyl refers to an alkynyl group having 2 to 8 carbon atoms, and includes, for example, but is not limited to, 1-propynyl, 2-propynyl and different butynyl, pentynyl and hexynyl isomers; and alkynyl also includes a group containing a plurality of triple bonds, such as 2,5-hexadiynyl.

Alkoxy refers to a group having an oxygen atom connected to the end of linear or branched alkyl, and the $C_{1-8}$ alkoxy refers to an alkoxy group having 1 to 8 carbon atoms, and includes, for example, but is not limited to, methoxy, ethoxy, n-propoxy, isopropoxy, n-butoxy, and the like.

Cycloalkoxy is a group containing an oxygen atom in cycloalkyl, and the $C_{1-8}$ cycloalkoxy refers to a cycloalkoxy group having 1 to 8 carbon atoms, and includes, for example, but is not limited to, cyclopropoxy, cyclobutoxy, and the like.

Alkylthio refers to a group having a sulfur atom connected to the end of alkyl, and includes, for example, but is not limited to, methylthio, ethylthio, n-propylthio, isopropylthio, tert-butylthio, and the like.

Alkylsulfinyl refers to a group having sulfinyl connected to the end of alkyl, and includes, for example, but is not limited to, methylsulfinyl, ethylsulfinyl, n-propylsulfinyl, isopropylsulfinyl, tert-butylsulfinyl, and the like.

Alkylsulfonyl refers to a group having sulfonyl connected to the end of alkyl, and includes, for example, but is not limited to, methylsulfonyl, ethylsulfonyl, n-propylsulfonyl, isopropylsulfonyl, tert-butylsulfonyl, and the like.

Haloalkyl, haloalkenyl, haloalkynyl, halocycloalkyl, haloalkoxy, halocycloalkoxy, haloalkylthio, haloalkylsulfinyl, and haloalkylsulfonyl respectively mean groups formed by substituting at least one hydrogen atom in alkyl, alkenyl, alkynyl, cycloalkyl, alkoxy, cycloalkoxy, alkylthio, alkylsulfinyl, and alkylsulfonyl with a halogen atom, and when there are two or more halogen atoms, the halogen atoms may be the same or different.

In the present disclosure, cycloalkyl substituted alkyl, halocycloalkyl substituted alkyl, cycloalkyl substituted haloalkyl, alkoxy substituted alkyl, haloalkoxy substituted alkyl, alkoxy substituted haloalkyl, cycloalkoxy substituted alkyl, halocycloalkoxy substituted alkyl, and cycloalkoxy substituted haloalkyl respectively mean that at least one hydrogen atom in alkyl is substituted by cycloalkyl, halocycloalkyl, alkoxy, haloalkoxy, cycloalkoxy, and halocycloalkoxy.

In the present disclosure, heteroatoms include, but are not limited to, O, S, and N atoms.

Other groups have similar definitions to those described above except that the substituent groups or the number of carbon atoms are different, which will not be described in detail.

Other terms in the present disclosure may be interpreted in a manner conventional in the art.

As previously described, a first aspect of the present disclosure provides a benzimidazole compound or a salt thereof, wherein the benzimidazole compound has a structure represented by a formula (I):

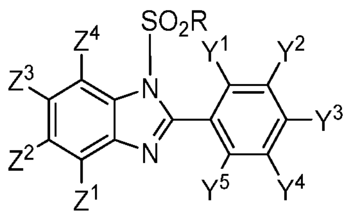

Formula (I);

wherein in the formula (I),

R is selected from substituted or unsubstituted $C_1$-$C_{10}$ alkyl, substituted or unsubstituted $C_3$-$C_{10}$ cycloalkyl, substituted or unsubstituted $C_2$-$C_{10}$ alkenyl, and substituted or unsubstituted $C_2$-$C_{10}$ alkynyl, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy;

$Y^1$, $Y^2$, $Y^3$, $Y^4$, and $Y^5$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsub-

stituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3- to 8-membered ring;

$Z^1$, $Z^2$, $Z^3$, and $Z^4$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

According to the present disclosure, the optionally means that a polycyclic ring structure may or may not be present.

According to the present disclosure, any two adjacent groups of $Y^1$, $Y^2$, $Y^3$, $Y^4$, and $Y^5$ form one group, and at least one group and the bonded benzene ring are cyclized with or without at least one heteroatom to form at least one 3- to 8-membered ring, which means that at least one combination of $Y^1$ and $Y^2$, $Y^2$ and $Y^3$, $Y^3$ and $Y^4$, and $Y^4$ and $Y^5$ and the bonded benzene ring form at least one 3- to 8-membered ring with or without at least one heteroatom. $Z^1$, $Z^2$, $Z^3$, and $Z^4$ have similar definitions thereto, which will not be repeated.

Preferably, in the formula (I),

R is selected from $C_1$-$C_{10}$ alkyl, halo $C_1$-$C_{10}$ alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkoxy substituted $C_1$-$C_{10}$ alkyl, halo $C_3$-$C_{10}$ cycloalkoxy substituted $C_1$-$C_{10}$ alkyl, $C_3$-$C_{10}$ cycloalkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy substituted $C_3$-$C_{10}$ cycloalkyl, halo $C_1$-$C_{10}$ alkoxy substituted $C_3$-$C_{10}$ cycloalkyl, $C_1$-$C_{10}$ alkoxy substituted halo $C_3$-$C_{10}$ cycloalkyl, $C_3$-$C_{10}$ cycloalkoxy substituted $C_3$-$C_{10}$ cycloalkyl, halo $C_3$-$C_{10}$ cycloalkoxy substituted $C_3$-$C_{10}$ cycloalkyl, $C_3$-$C_{10}$ cycloalkoxy substituted halo $C_3$-$C_{10}$ cycloalkyl, $C_2$-$C_{10}$ alkenyl, halo $C_2$-$C_{10}$ alkenyl, $C_2$-$C_{10}$ alkynyl, and halo $C_2$-$C_{10}$ alkynyl;

$Y^1$, $Y^2$, $Y^3$, $Y^4$, and $Y^5$ are each independently selected from H, halogen, CN, $NO_2$, $C_1$-$C_{10}$ alkyl, halo $C_1$-$C_{10}$ alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$

alkoxy, halo $C_1$-$C_{10}$ alkoxy, $C_1$-$C_{10}$ alkylthio, halo $C_1$-$C_{10}$ alkylthio, $C_1$-$C_{10}$ alkylsulfinyl, halo $C_1$-$C_{10}$ alkylsulfinyl, $C_1$-$C_{10}$ alkylsulfonyl, halo $C_1$-$C_{10}$ alkylsulfonyl, formyl, $C_1$-$C_{10}$ alkylcarbonyl, halo $C_1$-$C_{10}$ alkylcarbonyl, $C_1$-$C_{10}$ alkoxycarbonyl, halo $C_1$-$C_{10}$ alkoxycarbonyl, arylcarbonyl, aryloxycarbonyl, $C_2$-$C_6$ alkenyl, $C_2$-$C_6$ alkynyl, halo $C_2$-$C_6$ alkenyl, halo $C_2$-$C_6$ alkynyl, $C_2$-$C_6$ alkenyloxy, $C_2$-$C_6$ alkynyloxy, halo $C_2$-$C_6$ alkenyloxy, halo $C_2$-$C_6$ alkynyloxy, $C_1$-$C_{10}$ alkylcarbonyloxy, halo $C_1$-$C_{10}$ alkylcarbonyloxy, $C_1$-$C_{10}$ cyanoalkyl, $C_1$-$C_{10}$ cyanoalkoxy, $C_1$-$C_{10}$ alkyl substituted silyl, substituted or unsubstituted amino, aryl, aryl $C_1$-$C_6$ alkyl, aryloxy, aryl $C_1$-$C_6$ alkoxy, arylsulfonyl, arylsulfinyl, arylthio, aryl $C_1$-$C_6$ alkylsulfonyl, aryl $C_1$-$C_6$ alkylsulfinyl, aryl $C_1$-$C_6$ alkylthio, heterocyclyl, heterocyclyl $C_1$-$C_6$ alkyl, and heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3- to 8-membered ring; $Z^1$, $Z^2$, $Z^3$, and $Z^4$ are each independently selected from H, halogen, CN, $NO_2$, $C_1$-$C_{10}$ alkyl, halo $C_1$-$C_{10}$ alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy, halo $C_1$-$C_{10}$ alkoxy, $C_1$-$C_{10}$ alkylthio, halo $C_1$-$C_{10}$ alkylthio, $C_1$-$C_{10}$ alkylsulfinyl, halo $C_1$-$C_{10}$ alkylsulfinyl, $C_1$-$C_{10}$ alkylsulfonyl, halo $C_1$-$C_{10}$ alkylsulfonyl, formyl, $C_1$-$C_{10}$ alkylcarbonyl, halo $C_1$-$C_{10}$ alkylcarbonyl, $C_1$-$C_{10}$ alkoxycarbonyl, halo $C_1$-$C_{10}$ alkoxycarbonyl, arylcarbonyl, aryloxycarbonyl, $C_2$-$C_6$ alkenyl, $C_2$-$C_6$ alkynyl, halo $C_2$-$C_6$ alkenyl, halo $C_2$-$C_6$ alkynyl, $C_2$-$C_6$ alkenyloxy, $C_2$-$C_6$ alkynyloxy, halo $C_2$-$C_6$ alkenyloxy, halo $C_2$-$C_6$ alkynyloxy, $C_1$-$C_{10}$ alkylcarbonyloxy, halo $C_1$-$C_{10}$ alkylcarbonyloxy, $C_1$-$C_{10}$ cyanoalkyl, $C_1$-$C_{10}$ cyanoalkoxy, $C_1$-$C_{10}$ alkyl substituted silyl, substituted or unsubstituted amino, aryl, aryl $C_1$-$C_6$ alkyl, aryloxy, aryl $C_1$-$C_6$ alkoxy, arylsulfonyl, arylsulfinyl, arylthio, aryl $C_1$-$C_6$ alkylsulfonyl, aryl $C_1$-$C_6$ alkylsulfinyl, aryl $C_1$-$C_6$ alkylthio, heterocyclyl, heterocyclyl $C_1$-$C_6$ alkyl, and heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

Several particularly preferred specific embodiments of the benzimidazole compound of the present disclosure are provided below.

Specific embodiment 1

R is selected from $C_1$-$C_8$ alkyl, halo $C_1$-$C_8$ alkyl, $C_3$-$C_8$ cycloalkyl substituted $C_1$-$C_8$ alkyl, halo $C_3$-$C_8$ cycloalkyl substituted $C_1$-$C_8$ alkyl, $C_3$-$C_8$ cycloalkyl substituted halo $C_1$-$C_8$ alkyl, $C_1$-$C_8$ alkoxy substituted $C_1$-$C_8$ alkyl, halo $C_1$-$C_8$ alkoxy substituted $C_1$-$C_8$ alkyl, $C_1$-$C_8$ alkoxy substituted halo $C_1$-$C_8$ alkyl, $C_3$-$C_8$ cycloalkoxy substituted $C_1$-$C_8$ alkyl, halo $C_3$-$C_8$ cycloalkoxy substituted $C_1$-$C_8$ alkyl, $C_3$-$C_8$ cycloalkoxy substituted halo $C_1$-$C_8$ alkyl, $C_3$-$C_8$ cycloalkyl, halo $C_3$-$C_8$ cycloalkyl, $C_1$-$C_8$ alkyl substituted $C_3$-$C_8$ cycloalkyl, halo $C_1$-$C_8$ alkyl substituted $C_3$-$C_8$ cycloalkyl, $C_1$-$C_8$ alkyl substituted halo $C_3$-$C_8$ cycloalkyl, $C_1$-$C_8$ alkoxy substituted $C_3$-$C_8$ cycloalkyl, halo $C_1$-$C_8$ alkoxy substituted $C_3$-$C_8$ cycloalkyl, $C_1$-$C_8$ alkoxy substituted halo $C_3$-$C_8$ cycloalkyl, $C_3$-$C_8$ cycloalkoxy substituted $C_3$-$C_8$ cycloalkyl, halo $C_3$-$C_8$ cycloalkoxy substituted $C_3$-$C_8$ cycloalkyl, $C_3$-$C_8$ cycloalkoxy substituted halo $C_3$-$C_8$ cycloalkyl, $C_2$-$C_8$ alkenyl, halo $C_2$-$C_8$ alkenyl, $C_2$-$C_8$ alkynyl, and halo $C_2$-$C_8$ alkynyl;

$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_3$-$C_8$ cycloalkyl substituted $C_1$-$C_8$ alkyl, halo $C_3$-$C_8$ cycloalkyl substituted $C_1$-$C_8$ alkyl, $C_3$-$C_8$ cycloalkyl substituted halo $C_1$-$C_8$ alkyl, $C_1$-$C_8$ alkoxy substituted $C_1$-$C_8$ alkyl, halo $C_1$-$C_8$ alkoxy substituted $C_1$-$C_8$ alkyl, $C_1$-$C_8$ alkoxy substituted halo $C_1$-$C_8$ alkyl, $C_3$-$C_8$ cycloalkyl, halo $C_3$-$C_8$ cycloalkyl, $C_1$-$C_8$ alkyl substituted $C_3$-$C_8$ cycloalkyl, halo $C_1$-$C_8$ alkyl substituted $C_3$-$C_8$ cycloalkyl, $C_1$-$C_8$ alkyl substituted halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl;

$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_3$-$C_8$ cycloalkyl, halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$

are all H and R is ethyl, $Y^2$ and $Y^4$ are not simultaneously chlorine.

Specific embodiment 2

In the formula (I),

R is selected from $C_1$-$C_8$ alkyl, halo $C_1$-$C_8$ alkyl, $C_3$-$C_8$ cycloalkyl, halo $C_3$-$C_8$ cycloalkyl, $C_2$-$C_8$ alkenyl, halo $C_2$-$C_8$ alkenyl, $C_2$-$C_8$ alkynyl, and halo $C_2$-$C_8$ alkynyl;
$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_3$-$C_8$ cycloalkyl, halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl;
$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

Specific embodiment 3

In the formula (I),

R is selected from $C_1$-$C_8$ alkyl, halo $C_1$-$C_8$ alkyl, $C_3$-$C_8$ cycloalkyl, halo $C_3$-$C_8$ cycloalkyl, $C_2$-$C_5$ alkenyl, and $C_2$-$C_5$ alkynyl;
$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_1$-$C_8$ alkoxy, and halo $C_1$-$C_8$ alkoxy;
$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_1$-$C_8$ alkoxy, and halo $C_1$-$C_8$ alkoxy; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

Specific embodiment 4

In the formula (I),

R is selected from methyl, ethyl, n-propyl, isopropyl, cyclopropyl, n-butyl, sec-butyl, isobutyl, tert-butyl, allyl, propargyl, and $CF_3$;
$Y^1$, $Y^2$, $Y^3$, $Y^4$, and $Y^5$ are each independently selected from H, F, Cl, Br, I, CN, $NO_2$, $CH_3$, $CF_3$, $OCH_3$, $OCF_3$, $OCH_2CF_3$, $OCH_2CF_2CF_3$, $CF_2Cl$, $CFCl_2$, and $CCl_3$;
$Z^1$, $Z^2$, $Z^3$, and $Z^4$ are each independently selected from H, F, Cl, Br, I, CN, $NO_2$, $CH_3$, $CF_3$, $OCF_3$, ethyl, n-propyl, isopropyl, n-butyl, sec-butyl, isobutyl, and tert-butyl; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

Specific embodiment 5

In the formula (I),

R is selected from methyl, ethyl, n-propyl, isopropyl, n-butyl, sec-butyl, and isobutyl;
$Y^1$, $Y^2$, $Y^3$, $Y^4$, and $Y^5$ are each independently selected from H, F, Cl, Br, CN, $NO_2$, $OCF_3$, $CH_3$, $CF_3$, $OCH_3$, $OCH_2CF_3$, and $OCH_2CF_2CF_3$;
$Z^1$, $Z^2$, $Z^3$, and $Z^4$ are each independently selected from H, F, Cl, Br, and $CH_3$; and when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

Specific embodiment 6

The benzimidazole compound is selected from at least one of compounds shown in Table 1:

Table 1

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-1 | $CH_3$ | H | F | H | H | H | H | H | H | H |
| 1-2 | $CH_3$ | H | Cl | H | H | H | H | H | H | H |
| 1-3 | $CH_3$ | H | Br | H | H | H | H | H | H | H |
| 1-4 | $CH_3$ | H | I | H | H | H | H | H | H | H |
| 1-5 | $CH_3$ | H | $CH_3$ | H | H | H | H | H | H | H |
| 1-6 | $CH_3$ | H | $OCH_3$ | H | H | H | H | H | H | H |
| 1-7 | $CH_3$ | H | $CF_3$ | H | H | H | H | H | H | H |
| 1-8 | $CH_3$ | H | $NO_2$ | H | H | H | H | H | H | H |
| 1-9 | $CH_3$ | H | CN | H | H | H | H | H | H | H |
| 1-10 | $CH_3$ | H | $OCF_3$ | H | H | H | H | H | H | H |
| 1-11 | $CH_3$ | H | $CF_3$ | $CH_3$ | H | H | H | H | H | H |
| 1-12 | $CH_3$ | H | $CF_3$ | $OCH_3$ | H | H | H | H | H | H |
| 1-13 | $CH_3$ | H | $CF_3$ | F | H | H | H | H | H | H |
| 1-14 | $CH_3$ | H | $CF_3$ | Cl | H | H | H | H | H | H |
| 1-15 | $CH_3$ | H | $CF_3$ | Br | H | H | H | H | H | H |
| 1-16 | $CH_3$ | H | $CF_3$ | I | H | H | H | H | H | H |
| 1-17 | $CH_3$ | H | F | F | F | H | H | H | H | H |
| 1-18 | Et | H | F | H | H | H | H | H | H | H |
| 1-19 | Et | H | Cl | H | H | H | H | H | H | H |
| 1-20 | Et | H | Br | H | H | H | H | H | H | H |
| 1-21 | Et | H | $CH_3$ | H | H | H | H | H | H | H |
| 1-22 | Et | H | $OCH_3$ | H | H | H | H | H | H | H |
| 1-23 | Et | H | $CF_3$ | H | H | H | H | H | H | H |
| 1-24 | Et | H | $NO_2$ | H | H | H | H | H | H | H |
| 1-25 | Et | H | CN | H | H | H | H | H | H | H |
| 1-26 | Et | H | $OCF_3$ | H | H | H | H | H | H | H |
| 1-27 | Et | H | $CF_3$ | F | H | H | H | H | H | H |
| 1-28 | Et | H | $CF_3$ | Cl | H | H | H | H | H | H |
| 1-29 | Et | H | $CF_3$ | Br | H | H | H | H | H | H |
| 1-30 | Et | H | $CF_3$ | $CH_3$ | H | H | H | H | H | H |
| 1-31 | Et | H | $CF_3$ | $OCH_2CF_3$ | H | H | H | H | H | H |
| 1-32 | Et | H | $CF_3$ | $OCH_2CF_2CF_3$ | H | H | H | H | H | H |
| 1-33 | Et | H | F | F | H | H | H | H | H | H |
| 1-34 | Et | H | Cl | F | H | H | H | H | H | H |
| 1-35 | Et | H | Br | F | H | H | H | H | H | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-36 | Et | H | F | Cl | H | H | H | H | H | H |
| 1-37 | Et | H | Cl | Cl | H | H | H | H | H | H |
| 1-38 | Et | H | Br | Cl | H | H | H | H | H | H |
| 1-39 | Et | H | F | Br | H | H | H | H | H | H |
| 1-40 | Et | H | Cl | Br | H | H | H | H | H | H |
| 1-41 | Et | H | F | $CH_3$ | H | H | H | H | H | H |
| 1-42 | Et | H | F | $OCH_3$ | H | H | H | H | H | H |
| 1-43 | Et | H | F | $OCH_2CF_3$ | H | H | H | H | H | H |
| 1-44 | Et | H | $CH_3$ | $CH_3$ | H | H | H | H | H | H |
| 1-45 | Et | H | F | H | F | H | H | H | H | H |
| 1-46 | Et | H | $CH_3$ | H | $CH_3$ | H | H | H | H | H |
| 1-47 | Et | H | $CF_3$ | H | $CF_3$ | H | H | H | H | H |
| 1-48 | Et | F | H | F | F | H | H | H | H | H |
| 1-49 | Et | H | F | F | F | H | H | H | H | H |
| 1-50 | Et | H | F | F | H | H | H | F | F | H |
| 1-51 | Et | H | Cl | F | H | H | H | F | F | H |
| 1-52 | Et | H | Br | F | H | H | H | F | F | H |
| 1-53 | Et | H | F | Cl | H | H | H | F | F | H |
| 1-54 | Et | H | Cl | Cl | H | H | H | F | F | H |
| 1-55 | Et | H | Br | Cl | H | H | H | F | F | H |
| 1-56 | Et | H | $CF_3$ | F | H | H | H | F | F | H |
| 1-57 | Et | H | $CF_3$ | Cl | H | H | H | F | F | H |
| 1-58 | Et | H | $CF_3$ | $CH_3$ | H | H | H | F | F | H |
| 1-59 | Et | H | F | $OCH_3$ | H | H | H | F | F | H |
| 1-60 | Et | F | H | F | F | H | H | F | F | H |
| 1-61 | Et | H | F | F | F | H | H | F | F | H |
| 1-62 | Et | H | F | F | H | H | H | Cl | Cl | H |
| 1-63 | Et | H | Cl | F | H | H | H | Cl | Cl | H |
| 1-64 | Et | H | Br | F | H | H | H | Cl | Cl | H |
| 1-65 | Et | H | F | Cl | H | H | H | Cl | Cl | H |
| 1-66 | Et | H | Cl | Cl | H | H | H | Cl | Cl | H |
| 1-67 | Et | H | Br | Cl | H | H | H | Cl | Cl | H |
| 1-68 | Et | H | $CF_3$ | F | H | H | H | Cl | Cl | H |
| 1-69 | Et | H | $CF_3$ | Cl | H | H | H | Cl | Cl | H |
| 1-70 | Et | H | $CF_3$ | $CH_3$ | H | H | H | Cl | Cl | H |
| 1-71 | Et | H | F | $OCH_3$ | H | H | H | Cl | Cl | H |
| 1-72 | Et | F | H | F | F | H | H | Cl | Cl | H |
| 1-73 | Et | H | F | F | F | H | H | Cl | Cl | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-74 | Et | H | $CF_3$ | F | H | H | H | $CH_3$ | $CH_3$ | H |
| 1-75 | Et | H | $CF_3$ | Cl | H | H | H | $CH_3$ | $CH_3$ | H |
| 1-76 | Et | H | $CF_3$ | Br | H | H | H | $CH_3$ | $CH_3$ | H |
| 1-77 | Et | H | $CF_3$ | $CH_3$ | H | H | H | $CH_3$ | $CH_3$ | H |
| 1-78 | Et | H | $CH_3$ | $CH_3$ | H | H | H | $CH_3$ | $CH_3$ | H |
| 1-79 | Et | H | F | F | H | H | H | $CH_3$ | $CH_3$ | H |
| 1-80 | Et | H | Cl | F | H | H | H | $CH_3$ | $CH_3$ | H |
| 1-81 | Et | H | $CF_3$ | H | $CF_3$ | H | H | $CH_3$ | $CH_3$ | H |
| 1-82 | Et | F | H | F | F | H | H | $CH_3$ | $CH_3$ | H |
| 1-83 | Et | H | F | F | F | H | H | $CH_3$ | $CH_3$ | H |
| 1-84 | Et | F | F | F | F | F | H | H | H | H |
| 1-85 | Et | F | F | F | F | F | H | F | F | H |
| 1-86 | Et | F | F | F | F | F | F | F | F | F |
| 1-87 | $CH_3$ | H | F | H | F | H | H | H | H | H |
| 1-88 | $CH_3$ | H | F | H | Cl | H | H | H | H | H |
| 1-89 | $CH_3$ | H | F | H | Br | H | H | H | H | H |
| 1-90 | $CH_3$ | H | F | H | I | H | H | H | H | H |
| 1-91 | $CH_3$ | H | F | H | $CH_3$ | H | H | H | H | H |
| 1-92 | $CH_3$ | H | F | H | $OCH_3$ | H | H | H | H | H |
| 1-93 | $CH_3$ | H | F | H | $CF_3$ | H | H | H | H | H |
| 1-94 | $CH_3$ | H | F | H | CN | H | H | H | H | H |
| 1-95 | $CH_3$ | H | F | H | $NO_2$ | H | H | H | H | H |
| 1-96 | $CH_3$ | H | F | H | $OCF_3$ | H | H | H | H | H |
| 1-97 | $CH_3$ | H | Br | H | Cl | H | H | H | H | H |
| 1-98 | $CH_3$ | H | Br | H | Br | H | H | H | H | H |
| 1-99 | $CH_3$ | H | Br | H | I | H | H | H | H | H |
| 1-100 | $CH_3$ | H | Br | H | $CH_3$ | H | H | H | H | H |
| 1-101 | $CH_3$ | H | Br | H | $OCH_3$ | H | H | H | H | H |
| 1-102 | $CH_3$ | H | Br | H | $CF_3$ | H | H | H | H | H |
| 1-103 | $CH_3$ | H | Br | H | CN | H | H | H | H | H |
| 1-104 | $CH_3$ | H | Br | H | $NO_2$ | H | H | H | H | H |
| 1-105 | $CH_3$ | H | Br | H | $OCF_3$ | H | H | H | H | H |
| 1-106 | $CH_3$ | H | I | H | Cl | H | H | H | H | H |
| 1-107 | $CH_3$ | H | I | H | Br | H | H | H | H | H |
| 1-108 | $CH_3$ | H | I | H | I | H | H | H | H | H |
| 1-109 | $CH_3$ | H | I | H | $CH_3$ | H | H | H | H | H |
| 1-110 | $CH_3$ | H | I | H | $OCH_3$ | H | H | H | H | H |
| 1-111 | $CH_3$ | H | I | H | $CF_3$ | H | H | H | H | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-112 | $CH_3$ | H | I | H | CN | H | H | H | H | H |
| 1-113 | $CH_3$ | H | I | H | $NO_2$ | H | H | H | H | H |
| 1-114 | $CH_3$ | H | I | H | $OCF_3$ | H | H | H | H | H |
| 1-115 | $CH_3$ | H | $CH_3$ | H | Cl | H | H | H | H | H |
| 1-116 | $CH_3$ | H | $CH_3$ | H | Br | H | H | H | H | H |
| 1-117 | $CH_3$ | H | $CH_3$ | H | I | H | H | H | H | H |
| 1-118 | $CH_3$ | H | $CH_3$ | H | $CH_3$ | H | H | H | H | H |
| 1-119 | $CH_3$ | H | $CH_3$ | H | $OCH_3$ | H | H | H | H | H |
| 1-120 | $CH_3$ | H | $CH_3$ | H | $CF_3$ | H | H | H | H | H |
| 1-121 | $CH_3$ | H | $CH_3$ | H | CN | H | H | H | H | H |
| 1-122 | $CH_3$ | H | $CH_3$ | H | $NO_2$ | H | H | H | H | H |
| 1-123 | $CH_3$ | H | $CH_3$ | H | $OCF_3$ | H | H | H | H | H |
| 1-124 | $CH_3$ | H | $OCH_3$ | H | Cl | H | H | H | H | H |
| 1-125 | $CH_3$ | H | $OCH_3$ | H | Br | H | H | H | H | H |
| 1-126 | $CH_3$ | H | $OCH_3$ | H | I | H | H | H | H | H |
| 1-127 | $CH_3$ | H | $OCH_3$ | H | $CH_3$ | H | H | H | H | H |
| 1-128 | $CH_3$ | H | $OCH_3$ | H | $OCH_3$ | H | H | H | H | H |
| 1-129 | $CH_3$ | H | $OCH_3$ | H | $CF_3$ | H | H | H | H | H |
| 1-130 | $CH_3$ | H | $OCH_3$ | H | CN | H | H | H | H | H |
| 1-131 | $CH_3$ | H | $OCH_3$ | H | $NO_2$ | H | H | H | H | H |
| 1-132 | $CH_3$ | H | $OCH_3$ | H | $OCF_3$ | H | H | H | H | H |
| 1-133 | $CH_3$ | H | $CF_3$ | H | Cl | H | H | H | H | H |
| 1-134 | $CH_3$ | H | $CF_3$ | H | Br | H | H | H | H | H |
| 1-135 | $CH_3$ | H | $CF_3$ | H | I | H | H | H | H | H |
| 1-136 | $CH_3$ | H | $CF_3$ | H | $CH_3$ | H | H | H | H | H |
| 1-137 | $CH_3$ | H | $CF_3$ | H | $OCH_3$ | H | H | H | H | H |
| 1-138 | $CH_3$ | H | $CF_3$ | H | $CF_3$ | H | H | H | H | H |
| 1-139 | $CH_3$ | H | $CF_3$ | H | CN | H | H | H | H | H |
| 1-140 | $CH_3$ | H | $CF_3$ | H | $NO_2$ | H | H | H | H | H |
| 1-141 | $CH_3$ | H | $CF_3$ | H | $OCF_3$ | H | H | H | H | H |
| 1-142 | $CH_3$ | H | CN | H | Cl | H | H | H | H | H |
| 1-143 | $CH_3$ | H | CN | H | Br | H | H | H | H | H |
| 1-144 | $CH_3$ | H | CN | H | I | H | H | H | H | H |
| 1-145 | $CH_3$ | H | CN | H | $CH_3$ | H | H | H | H | H |
| 1-146 | $CH_3$ | H | CN | H | $OCH_3$ | H | H | H | H | H |
| 1-147 | $CH_3$ | H | CN | H | $CF_3$ | H | H | H | H | H |
| 1-148 | $CH_3$ | H | CN | H | CN | H | H | H | H | H |
| 1-149 | $CH_3$ | H | CN | H | $NO_2$ | H | H | H | H | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-150 | $CH_3$ | H | CN | H | $OCF_3$ | H | H | H | H | H |
| 1-151 | $CH_3$ | H | $NO_2$ | H | Cl | H | H | H | H | H |
| 1-152 | $CH_3$ | H | $NO_2$ | H | Br | H | H | H | H | H |
| 1-153 | $CH_3$ | H | $NO_2$ | H | I | H | H | H | H | H |
| 1-154 | $CH_3$ | H | $NO_2$ | H | $CH_3$ | H | H | H | H | H |
| 1-155 | $CH_3$ | H | $NO_2$ | H | $OCH_3$ | H | H | H | H | H |
| 1-156 | $CH_3$ | H | $NO_2$ | H | $CF_3$ | H | H | H | H | H |
| 1-157 | $CH_3$ | H | $NO_2$ | H | CN | H | H | H | H | H |
| 1-158 | $CH_3$ | H | $NO_2$ | H | $NO_2$ | H | H | H | H | H |
| 1-159 | $CH_3$ | H | $NO_2$ | H | $OCF_3$ | H | H | H | H | H |
| 1-160 | $CH_3$ | H | $OCF_3$ | H | Cl | H | H | H | H | H |
| 1-161 | $CH_3$ | H | $OCF_3$ | H | Br | H | H | H | H | H |
| 1-162 | $CH_3$ | H | $OCF_3$ | H | I | H | H | H | H | H |
| 1-163 | $CH_3$ | H | $OCF_3$ | H | $CH_3$ | H | H | H | H | H |
| 1-164 | $CH_3$ | H | $OCF_3$ | H | $OCH_3$ | H | H | H | H | H |
| 1-165 | $CH_3$ | H | $OCF_3$ | H | $CF_3$ | H | H | H | H | H |
| 1-166 | $CH_3$ | H | $OCF_3$ | H | CN | H | H | H | H | H |
| 1-167 | $CH_3$ | H | $OCF_3$ | H | $NO_2$ | H | H | H | H | H |
| 1-168 | $CH_3$ | H | $OCF_3$ | H | $OCF_3$ | H | H | H | H | H |
| 1-169 | Et | H | F | H | Cl | H | H | H | H | H |
| 1-170 | Et | H | F | H | Br | H | H | H | H | H |
| 1-171 | Et | H | F | H | I | H | H | H | H | H |
| 1-172 | Et | H | F | H | $CH_3$ | H | H | H | H | H |
| 1-173 | Et | H | F | H | $OCH_3$ | H | H | H | H | H |
| 1-174 | Et | H | F | H | $CF_3$ | H | H | H | H | H |
| 1-175 | Et | H | F | H | CN | H | H | H | H | H |
| 1-176 | Et | H | F | H | $NO_2$ | H | H | H | H | H |
| 1-177 | Et | H | F | H | $OCF_3$ | H | H | H | H | H |
| 1-178 | Et | H | Br | H | Cl | H | H | H | H | H |
| 1-179 | Et | H | Br | H | Br | H | H | H | H | H |
| 1-180 | Et | H | Br | H | I | H | H | H | H | H |
| 1-181 | Et | H | Br | H | $CH_3$ | H | H | H | H | H |
| 1-182 | Et | H | Br | H | $OCH_3$ | H | H | H | H | H |
| 1-183 | Et | H | Br | H | $CF_3$ | H | H | H | H | H |
| 1-184 | Et | H | Br | H | CN | H | H | H | H | H |
| 1-185 | Et | H | Br | H | $NO_2$ | H | H | H | H | H |
| 1-186 | Et | H | Br | H | $OCF_3$ | H | H | H | H | H |
| 1-187 | Et | H | I | H | Cl | H | H | H | H | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-188 | Et | H | I | H | Br | H | H | H | H | H |
| 1-189 | Et | H | I | H | I | H | H | H | H | H |
| 1-190 | Et | H | I | H | $CH_3$ | H | H | H | H | H |
| 1-191 | Et | H | I | H | $OCH_3$ | H | H | H | H | H |
| 1-192 | Et | H | I | H | $CF_3$ | H | H | H | H | H |
| 1-193 | Et | H | I | H | CN | H | H | H | H | H |
| 1-194 | Et | H | I | H | $NO_2$ | H | H | H | H | H |
| 1-195 | Et | H | I | H | $OCF_3$ | H | H | H | H | H |
| 1-196 | Et | H | $CH_3$ | H | Cl | H | H | H | H | H |
| 1-197 | Et | H | $CH_3$ | H | Br | H | H | H | H | H |
| 1-198 | Et | H | $CH_3$ | H | I | H | H | H | H | H |
| 1-199 | Et | H | $CH_3$ | H | $OCH_3$ | H | H | H | H | H |
| 1-200 | Et | H | $CH_3$ | H | $CF_3$ | H | H | H | H | H |
| 1-201 | Et | H | $CH_3$ | H | CN | H | H | H | H | H |
| 1-202 | Et | H | $CH_3$ | H | $NO_2$ | H | H | H | H | H |
| 1-203 | Et | H | $CH_3$ | H | $OCF_3$ | H | H | H | H | H |
| 1-204 | Et | H | $OCH_3$ | H | Cl | H | H | H | H | H |
| 1-205 | Et | H | $OCH_3$ | H | Br | H | H | H | H | H |
| 1-206 | Et | H | $OCH_3$ | H | I | H | H | H | H | H |
| 1-207 | Et | H | $OCH_3$ | H | $CH_3$ | H | H | H | H | H |
| 1-208 | Et | H | $OCH_3$ | H | $OCH_3$ | H | H | H | H | H |
| 1-209 | Et | H | $OCH_3$ | H | $CF_3$ | H | H | H | H | H |
| 1-210 | Et | H | $OCH_3$ | H | CN | H | H | H | H | H |
| 1-211 | Et | H | $OCH_3$ | H | $NO_2$ | H | H | H | H | H |
| 1-212 | Et | H | $OCH_3$ | H | $OCF_3$ | H | H | H | H | H |
| 1-213 | Et | H | $CF_3$ | H | Cl | H | H | H | H | H |
| 1-214 | Et | H | $CF_3$ | H | Br | H | H | H | H | H |
| 1-215 | Et | H | $CF_3$ | H | I | H | H | H | H | H |
| 1-216 | Et | H | $CF_3$ | H | $CH_3$ | H | H | H | H | H |
| 1-217 | Et | H | $CF_3$ | H | $OCH_3$ | H | H | H | H | H |
| 1-218 | Et | H | $CF_3$ | H | CN | H | H | H | H | H |
| 1-219 | Et | H | $CF_3$ | H | $NO_2$ | H | H | H | H | H |
| 1-220 | Et | H | $CF_3$ | H | $OCF_3$ | H | H | H | H | H |
| 1-221 | Et | H | CN | H | Cl | H | H | H | H | H |
| 1-222 | Et | H | CN | H | Br | H | H | H | H | H |
| 1-223 | Et | H | CN | H | I | H | H | H | H | H |
| 1-224 | Et | H | CN | H | $CH_3$ | H | H | H | H | H |
| 1-225 | Et | H | CN | H | $OCH_3$ | H | H | H | H | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-226 | Et | H | CN | H | $CF_3$ | H | H | H | H | H |
| 1-227 | Et | H | CN | H | CN | H | H | H | H | H |
| 1-228 | Et | H | CN | H | $NO_2$ | H | H | H | H | H |
| 1-229 | Et | H | CN | H | $OCF_3$ | H | H | H | H | H |
| 1-230 | Et | H | $NO_2$ | H | Cl | H | H | H | H | H |
| 1-231 | Et | H | $NO_2$ | H | Br | H | H | H | H | H |
| 1-232 | Et | H | $NO_2$ | H | I | H | H | H | H | H |
| 1-233 | Et | H | $NO_2$ | H | $CH_3$ | H | H | H | H | H |
| 1-234 | Et | H | $NO_2$ | H | $OCH_3$ | H | H | H | H | H |
| 1-235 | Et | H | $NO_2$ | H | $CF_3$ | H | H | H | H | H |
| 1-236 | Et | H | $NO_2$ | H | CN | H | H | H | H | H |
| 1-237 | Et | H | $NO_2$ | H | $NO_2$ | H | H | H | H | H |
| 1-238 | Et | H | $NO_2$ | H | $OCF_3$ | H | H | H | H | H |
| 1-239 | Et | H | $OCF_3$ | H | Cl | H | H | H | H | H |
| 1-240 | Et | H | $OCF_3$ | H | Br | H | H | H | H | H |
| 1-241 | Et | H | $OCF_3$ | H | I | H | H | H | H | H |
| 1-242 | Et | H | $OCF_3$ | H | $CH_3$ | H | H | H | H | H |
| 1-243 | Et | H | $OCF_3$ | H | $OCH_3$ | H | H | H | H | H |
| 1-244 | Et | H | $OCF_3$ | H | $CF_3$ | H | H | H | H | H |
| 1-245 | Et | H | $OCF_3$ | H | CN | H | H | H | H | H |
| 1-246 | Et | H | $OCF_3$ | H | $NO_2$ | H | H | H | H | H |
| 1-247 | Et | H | $OCF_3$ | H | $OCF_3$ | H | H | H | H | H |
| 1-248 | n-Pr | H | $CF_3$ | F | H | H | H | H | H | H |
| 1-249 | n-Bu | H | $CF_3$ | F | H | H | H | H | H | H |
| 1-250 | $CH_3$ | H | $CF_3$ | F | H | H | H | H | H | H |
| 1-251 | $CF_3$ | H | $CF_3$ | H | $CF_3$ | H | H | H | H | H |
| 1-252 | $CH_3$ | H | $CF_3$ | H | $CF_3$ | H | H | H | H | H |
| 1-253 | Cyclopropyl | H | $CF_3$ | F | H | H | H | H | H | H |
| 1-254 | n-Pr | H | $CF_3$ | H | $CF_3$ | H | H | H | H | H |
| 1-255 | n-Bu | H | $CF_3$ | H | $CF_3$ | H | H | H | H | H |
| 1-256 | Cyclopropyl | H | $CF_3$ | H | $CF_3$ | H | H | H | H | H |
| 1-257 | Et | H | $CF_3$ | H | $CF_3$ | H | H | F | F | H |
| 1-258 | Et | H | Cl | $CH_3$ | Cl | H | H | H | H | H |
| 1-259 | Et | H | $CF_3$ | H | $CF_3$ | H | H | t-Bu | H | H |
| 1-260 | Et | H | $CF_3$ | H | $CF_3$ | H | H | H | $OCF_3$ | H |
| 1-261 | Et | H | $CF_3$ | H | $CF_3$ | H | H | H | F | H |
| 1-262 | Et | H | $CF_3$ | H | $CF_3$ | H | $CH_3$ | H | H | H |
| 1-263 | Et | H | $CF_3$ | H | $CF_3$ | H | H | $CH_3$ | $CH_3$ | H |

(continued)

| Compound No. | R | $Y^1$ | $Y^2$ | $Y^3$ | $Y^4$ | $Y^5$ | $Z^1$ | $Z^2$ | $Z^3$ | $Z^4$ |
|---|---|---|---|---|---|---|---|---|---|---|
| 1-264 | Et | H | $CF_3$ | H | $CF_3$ | H | H | $CH_3$ | H | H |
| 1-265 | Et | H | $CF_3$ | H | $CF_3$ | H | H | Cl | Cl | H |
| 1-266 | Et | H | $CF_3$ | H | $CF_3$ | H | H | H | $CF_3$ | H |
| 1-267 | Et | H | $CH_3$ | H | $CH_3$ | H | H | H | H | H |

Specific embodiment 7

The benzimidazole compound is selected from at least one of a compound 1-25, a compound 1-26, a compound 1-27, a compound 1-28, a compound 1-29, a compound 1-30, a compound 1-31, a compound 1-32, a compound 1-33, a compound 1-34, a compound 1-37, a compound 1-38, a compound 1-40, a compound 1-43, a compound 1-47, a compound 1-49, a compound 1-50, a compound 1-51, a compound 1-52, a compound 1-54, a compound 1-55, a compound 1-56, a compound 1-57, a compound 1-60, a compound 1-61, a compound 1-62, a compound 1-68, a compound 1-69, a compound 1-72, a compound 1-73, a compound 1-75, a compound 1-76, a compound 1-81, a compound 1-133, a compound 1-174, a compound 1-186, a compound 1-213, a compound 1-214, a compound 1-218, a compound 1-235, a compound 1-239, a compound 1-254, a compound 1-255, a compound 1-257, a compound 1-261, a compound 1-264, and a compound 1-265.

Specific embodiment 8

The benzimidazole compound is selected from at least one of a compound 1-26, a compound 1-27, a compound 1-28, a compound 1-29, a compound 1-30, a compound 1-31, a compound 1-32, a compound 1-34, a compound 1-37, a compound 1-38, a compound 1-47, a compound 1-49, a compound 1-56, a compound 1-57, a compound 1-61, a compound 1-68, a compound 1-69, a compound 1-73, a compound 1-133, a compound 1-174, a compound 1-186, a compound 1-213, a compound 1-214, a compound 1-218, a compound 1-235, a compound 1-239, a compound 1-254, a compound 1-255, a compound 1-257, a compound 1-261, a compound 1-264, and a compound 1-265.

Specific embodiment 9

The benzimidazole compound is selected from at least one of a compound 1-27, a compound 1-28, a compound 1-47, a compound 1-56, a compound 1-57, a compound 1-133, a compound 1-174, a compound 1-186, a compound 1-213, a compound 1-214, a compound 1-218, a compound 1-235, a compound 1-239, a compound 1-254, a compound 1-255, a compound 1-257, a compound 1-261, a compound 1-264, and a compound 1-265.

The inventors of the present disclosure found that the benzimidazole compounds or salts thereof in several preferred specific embodiments provided above have better insecticidal and acaricidal effects, and in particular, the benzimidazole compounds or salts thereof in the preferred specific embodiments 7-9, in particular, the compounds in preferred specific embodiment 9 have superior insecticidal and acaricidal effects, and can have excellent insecticidal and acaricidal effects when used at a low concentration (e.g. 6.25 mg/L).

Furthermore, the present disclosure provides performance data (NMR) of some of the compounds, as shown in Table 2:

Table 2

| Compound No. | $^1H$ NMR (400 MHz, $CDCl_3$) | LC-MS ($M+H^+$) |
|---|---|---|
| 1-20 | δ 8.04-7.96 (m, 1H), 7.92 (t, 1H), 7.88-7.80 (m, 1H), 7.76-7.62 (m, 2H), 7.52-7.41 (m, 2H), 7.37 (t, 1H), 3.17 (q, 2H), 1.08 (t, 3H). | 365.2, 367.2 |
| 1-26 | δ 8.04-7.98 (m, 1H), 7.88-7.81 (m, 1H), 7.70 (d, 1H), 7.64 (s, 1H), 7.53 (t, 1H), 7.50-7.44 (m, 2H), 7.41 (d, 1H), 3.18 (q, 2H), 1.07 (t, 3H). | 371.3 |
| 1-27 | δ 8.07 (d, 1H), 8.00 (m, 2H), 7.89-7.81 (m, 1H), 7.5-7.42 (m, 2H), 7.34 (t, 1H), 3.17 (q, 2H), 1.06 (t, 3H). | 373.3 |

(continued)

| Compound No. | $^1$H NMR (400 MHz, $CDCl_3$) | LC-MS (M+H$^+$) |
|---|---|---|
| 1-28 | δ 8.13 (d, 1H), 8.03-7.97 (m, 1H), 7.94-7.79 (m, 2H), 7.64 (d, 1H), 7.54-7.42 (m, 2H), 3.18 (q, 2H), 1.06 (t, 3H). | 389.3, 391.2 |
| 1-30 | δ 8.07-7.96 (m, 2H), 7.88-7.80 (m, 2H), 7.52-7.38 (m, 3H), 3.16 (q, 2H), 2.58 (s, 3H), 1.06 (t, 3H). | 369.3 |
| 1-31 | δ 8.09 (d, 1H), 8.04-7.93 (m, 2H), 7.89-7.77 (m, 1H), 7.53-7.42 (m, 2H), 7.09 (d, 1H), 4.53 (q, 2H), 3.15 (q, 2H), 1.05 (t, 3H). | 453.3 |
| 1-32 | δ 8.09 (d, 1H), 8.03-7.94 (m, 2H), 7.88-7.80 (m, 1H), 7.53-7.42 (m, 2H), 7.09 (d, 1H), 4.58 (t, 2H), 3.15 (q, 2H), 1.05 (t, 3H). | 503.2 |
| 1-34 | δ 8.04-7.95 (m, 1H), 7.90-7.79 (m, 2H), 7.67 (m, 1H), 7.52-7.43 (m, 2H), 7.25 (t, 1H), 3.17 (q, 2H), 1.06 (t, 3H). | 339.3, 341.3 |
| 1-35 | δ 8.06-7.95 (m, 2H), 7.8-7.78 (m, 1H), 7.72 (m, 1H), 7.52-7.40 (m, 2H), 7.25 (t, 1H), 3.16 (q, 2H), 1.06 (t, 3H). | 383.3, 385.2 |
| 1-36 | δ 8.04-7.95 (m, 1H), 7.88-7.79 (m, 1H), 7.59 (d, 1H), 7.55-7.40 (m, 4H), 3.16 (q, 2H), 1.06 (t, 3H). | 339.3, 341.3 |
| 1-37 | δ 8.04-7.96 (m, 1H), 7.89 (d, 1H), 7.87-7.78 (m, 1H), 7.60 (m, 2H), 7.52-7.42 (m, 2H), 3.17 (q, 2H), 1.06 (t, 3H). | 355.2, 357.2 |
| 1-38 | δ 8.06 (d, 1H), 8.02-7.96 (m, 1H), 7.87-7.79 (m, 1H), 7.67 (dd, 1H), 7.57 | 399.2, 401.2, |
| | (d, 1H), 7.51-7.43 (m, 2H), 3.17 (q, 2H), 1.06 (t, 3H). | 403.2 |
| 1-39 | δ 8.04-7.96 (m, 1H), 7.88-7.81 (m, 1H), 7.68 (dd, 1H), 7.56 (dd, 1H), 7.51-7.41 (m, 3H), 3.17 (q, 2H), 1.06 (t, 3H). | 383.1, 385.1 |
| 1-40 | δ 8.03-7.95 (m, 1H), 7.89 (d, 1H), 7.87-7.81 (m, 1H), 7.74 (d, 1H), 7.53 (dd, 1H), 7.51-7.43 (m, 2H), 3.17 (q, 2H), 1.06 (t, 3H). | 399.1, 401.1, 403.1 |
| 1-42 | δ 8.05-7.95 (m, 1H), 7.82 (m, 1H), 7.56 (m, 2H), 7.50-7.37 (m, 2H), 7.06 (t, 1H), 3.97 (s, 3H), 3.14 (q, 2H), 1.05 (t, 3H). | 335.3 |
| 1-47 | δ 8.26 (s, 2H), 8.05 (s, 1H), 8.03-7.97 (m, 1H), 7.93-7.84 (m, 1H), 7.5-7.47 (m, 2H), 3.20 (q, 2H), 1.07 (t, 3H). | 423.0 |
| 1-48 | δ 7.97-7.88 (m, 1H), 7.88-7.81 (m, 1H), 7.54-7.41 (m, 3H), 7.07 (td, 1H), 3.39 (q, 2H), 1.26 (t, 3H). | 341.3 |
| 1-49 | δ 8.04-7.93 (m, 1H), 7.83 (m, 1H), 7.55-7.37 (m, 4H), 3.19 (q, 2H), 1.07 (t, 3H). | 341.3 |
| 1-51 | δ 7.92-7.78 (m, 2H), 7.71-7.55 (m, 2H), 7.28 (t, 1H), 3.15 (q, 2H), 1.10 (t, 3H). | 375.2, 377.2 |
| 1-52 | δ 7.99 (dd, 1H), 7.87 (dd, 1H), 7.71 (ddd, 1H), 7.61 (dd, 1H), 7.26 (t, 1H), 3.15 (q, 2H), 1.10 (t, 3H). | 419.1, 421.1 |
| 1-55 | δ 8.03 (d, 1H), 7.87 (dd, 1H), 7.69-7.55 (m, 3H), 3.15 (q, 2H), 1.10 (t, 3H). | 435.1, 437.1, 439.1 |
| 1-56 | δ 8.04 (d, 1H), 8.01-7.92 (m, 1H), 7.87 (dd, 1H), 7.62 (dd, 1H), 7.35 (t, 1H), 3.15 (q, 2H), 1.10 (t, 3H). | 409.2 |
| 1-57 | δ 8.11 (s, 1H), 7.93-7.80 (m, 2H), 7.70-7.55 (m, 2H), 3.16 (qd, J = 7.1, 3.0 Hz, 2H), 1.35-0.80 (m, 3H). | 425.1, 427.1 |
| 1-58 | δ 8.00 (s, 1H), 7.94-7.78 (m, 2H), 7.61 (dd, 1H), 7.42 (d, 1H), 3.14 (q, 2H), 2.57 (s, 3H), 1.10 (t, 3H). | 405.3 |
| 1-60 | δ 7.78 (dd, 1H), 7.63 (dd, 1H), 7.44 (td, 1H), 7.09 (td, 1H), 3.38 (q, 2H), 1.30 (t, 3H). | 377.2 |
| 1-61 | δ 7.86 (dd, 1H), 7.61 (dd, 1H), 7.44 (t, 2H), 3.18 (q, 2H), 1.12 (t, 3H). | 377.2 |

(continued)

| Compound No. | $^1$H NMR (400 MHz, $CDCl_3$) | LC-MS ($M+H^+$) |
|---|---|---|
| 1-62 | δ 8.14 (s, 1H), 7.91 (s, 1H), 7.70-7.58 (m, 1H), 7.58-7.48 (m, 1H), 7.37-7.24 (m, 1H), 3.16 (q, 2H), 1.10 (t, 3H). | 391.2, 393.2 |
| 1-65 | δ 8.14 (s, 1H), 7.92 (s, 1H), 7.64-7.47 (m, 3H), 3.17 (q, 2H), 1.11 (t, 3H). | 407.2, 409.1, 411.2 |
| 1-66 | δ 8.14 (s, 1H), 7.91 (s, 1H), 7.87 (d, 1H), 7.66-7.55 (m, 2H), 3.16 (q, 2H), 1.11 (t, 3H). | 423.0, 425.0, 427.1 |
| 1-67 | δ 8.13 (s, 1H), 8.04 (d, 1H), 7.91 (s, 1H), 7.66 (dd, 1H), 7.58 (d, 1H), 3.16 (q, 2H), 1.11 (t, 3H). | 467.0, 469.0, 471.0 |
| 1-68 | δ 8.14 (s, 1H), 8.09-8.01 (m, 1H), 8.01-7.95 (m, 1H), 7.93 (s, 1H), 7.35 (t, 1H), 3.16 (q, 2H), 1.10 (t, 3H). | 441.2, 443.2 |
| 1-69 | δ 8.12 (d, 2H), 8.00-7.85 (m, 2H), 7.68 (d, 1H), 3.17 (q, 2H), 1.11 (t, 3H). | 457.1, 459.1, 461.1 |
| 1-70 | δ 8.15 (s, 1H), 8.01 (s, 1H), 7.92 (s, 1H), 7.83 (d, 1H), 7.46 (d, 1H), 3.14 (q, 2H), 2.59 (s, 3H), 1.10 (t, 3H). | 437.2, 439.2 |
| 1-71 | δ 8.15 (s, 1H), 7.89 (s, 1H), 7.60-7.48 (m, 2H), 7.07 (t, 1H), 3.98 (s, 3H), | 403.3, 405.2 |
| | 3.13 (q, 2H), 1.08 (t, 3H). | |
| 1-72 | δ 8.05 (s, 1H), 7.94 (s, 1H), 7.51-7.38 (m, 1H), 7.09 (td, 1H), 3.39 (q, 2H), 1.32 (t, 3H). | 409.2, 411.2 |
| 1-73 | δ 8.13 (s, 1H), 7.92 (s, 1H), 7.49-7.39 (m, 2H), 3.19 (q, 2H), 1.12 (t, 3H). | 409.2, 411.2 |
| 1-111 | $^1$H NMR (400 MHz, $CDCl_3$) δ 8.03-7.97 (m, 1H), 7.97-7.94 (m, 1H), 7.94 (s, 1H), 7.89-7.82 (m, 1H), 7.77 (d, J = 17.1 Hz, 1H), 7.55-7.45 (m, 2H), 3.14-2.82 (m, 3H). | 375.3, 377.3 |
| 1-133 | $^1$H NMR (400 MHz, $CDCl_3$) δ 8.03-7.97 (m, 1H), 7.97-7.94 (m, 1H), 7.94 (s, 1H), 7.89-7.82 (m, 1H), 7.77 (d, J = 17.1 Hz, 1H), 7.55-7.45 (m, 2H), 3.14-2.82 (m, 3H) | 375.33, 377.35 |
| 1-134 | $^1$H NMR (400 MHz, $CDCl_3$) δ 8.10 (d, J = 12.6 Hz, 1H), 8.02-8.00 (m,1H), 7.98 (d, J = 2.1 Hz, 1H), 7.94 (s, 1H), 7.90-7.82 (m, 1H), 7.56-7.45 (m, 2H), 3.35-2.57 (m, 3H). | 419.2, 421.2 |
| 1-174 | $^l$HNMR (400 MHz, $CDCl_3$) δ 8.03-7.93 (m, 1H), 7.85 (dd, J = 7.4, 3.9 Hz, 2H), 7.68 (t, J = 10.4 Hz, 1H), 7.55-7.40 (m, 3H), 3.67-2.87 (m, 2H), 1.26-0.81 (m, 3H). | 373.36 |
| 1-175 | $^1$H NMR (400 MHz, $CDCl_3$) δ 7.98 (dd, J = 17.3, 12.1 Hz, 1H), 7.93 (s, 2H), 7.88-7.83 (m, 1H), 7.74 (t, J = 16.8 Hz, 1H), 7.52 (t, J = 7.7 Hz, 2H), 3.48-2.75 (m, 2H), 1.45-0.74 (m, 3H). | 330.5 |
| 1-173 | $^1$H NMR (400 MHz, $CDCl_3$) δ 8.05-7.95 (m, 1H), 7.89-7.80 (m, 1H), 7.80 (s, 1H), 7.59 (d, J = 5.2 Hz, 1H), 7.49-7.40 (m, 2H), 7.20 (dd, J = 6.2, 3.1 Hz, 1H), 3.30-2.86 (m, 2H), 1.20-1.01 (m, 3H). | 335.57 |
| 1-179 | $^1$H NMR (400 MHz, $CDCl_3$) δ 8.02-7.95 (m, 1H), 7.84 (s, 2H), 7.85-7.81 (m, 2H), 7.51-7.42 (m, 2H), 3.46-2.91 (m, 2H), 1.26-0.84 (m, 3H). | 443.10 445.08 447.05 |
| 1-182 | $^1$H NMR (400 MHz, $CDCl_3$) δ 8.01-7.96 (m, 1H), 7.87-7.82 (m, 1H), 7.81 (s, 1H), 7.72-7.69 (m, 1H), 7.69 (s, 1H), 7.51-7.44 (m, 2H), 3.42-3.05 (m, 2H), 1.17-0.72 (m, 3H). | 399.18 401.17 403.13 |
| 1-186 | $^1$H NMR (400 MHz, $CDCl_3$) δ 8.02-7.95 (m, 1H), 7.90-7.81 (m, 2H), 7.59 (s, 1H), 7.57 (s, 1H), 7.52-7.44 (m, 2H), 3.48-2.99 (m, 2H), 1.50-0.67 (m, 3H). | 451.24 |
| 1-211 | $^1$H NMR (400 MHz, $CDCl_3$) δ 8.05-7.96 (m, 1H), 7.87-7.79 (m, 1H), 7.48-7.40 (m, 2H), 7.37 (s, 2H), 7.20-7.10 (m, 1H), 3.22-3.02 (m, 2H), 2.37 (d, J = 15.8 Hz, 6H), 2.23 (s, 1H), 1.63-1.44 (m, 1H), 1.19-0.91 (m, 3H). | 315.36 |

(continued)

| Compound No. | $^1$H NMR (400 MHz, $CDCl_3$) | LC-MS (M+H$^+$) |
|---|---|---|
| 1-213 | $^I$HNMR (400 MHz, $CDCl_3$) δ 8.56 (d, J = 13.6 Hz, 1H), 7.98 (dd, J = 17.1, 7.4 Hz, 2H), 7.86 (dt, J = 18.7, 9.3 Hz, 2H), 7.50 (dd, J = 5.7, 3.3 Hz, 1H), 7.29-7.19 (m, 1H), 3.38-2.86 (m, 2H), 1.22-0.69 (m, 3H). | 389.24, 391.23 |
| 1-214 | $^1$H NMR (400 MHz, $CDCl_3$) δ 8.12 (s, 1H), 8.03-7.96 (m, 2H), 7.93 (s, 1H), 7.88-7.82 (m, 1H), 7.54-7.46 (m, 2H), 3.11 (dq, J = 70.4, 7.4 Hz, 2H), 1.15-0.90 (m, 3H). | 433.2 435.2 |
| 1-218 | $^1$H NMR (400 MHz, $CDCl_3$) δ 8.28 (d, J = 6.6 Hz, 2H), 8.07 (s, 1H), 8.02-7.95 (m, 1H), 7.92-7.82 (m, 1H), 7.59-7.49 (m, 2H), 3.51-2.87 (m, 2H), 1.51-0.66 (m, 3H). | 380.3 |
| 1-237 | $^1$H NMR (400 MHz, $CDCl_3$) δ 9.19 (s, 1H), 8.98 (s, 2H), 7.98 (t, J = 13.6 | 373.0 |
| | Hz, 1H), 7.94 (s, 1H), 7.62-7.46 (m, 2H), 3.28 (q, J = 7.3 Hz, 2H), 1.21-0.96 (m, 3H). | |
| 1-235 | $^1$H NMR (400 MHz, $CDCl_3$) δ 8.81 (d, J = 27.3 Hz, 1H), 8.70-8.59 (m, 1H), 8.39 (s, 1H), 8.06-7.95 (m, 1H), 7.89 (dt, J = 6.5, 2.7 Hz, 1H), 7.61-7.46 (m, 2H), 3.57-2.85 (m, 2H), 1.17-0.90 (m, 3H). | 400.3 |
| 1-248 | $^1$H NMR (400 MHz, $CDCl_3$) δ 8.06 (dd, J = 6.6, 1.8 Hz, 1H), 8.03-7.93 (m, 2H), 7.89-7.80 (m, 1H), 7.53-7.42 (m, 2H), 7.33 (t, J = 9.2 Hz, 1H), 3.34-2.80 (m, 2H), 1.68-1.39 (m, 2H), 0.87 (t, J = 7.4 Hz, 3H). | 387.0 |
| 1-249 | $^1$H NMR (400 MHz, $CDCl_3$) δ 8.06 (d, J = 7.6 Hz, 1H), 8.00 (s, 2H), 7.87 (s, 1H), 7.50 (s, 2H), 7.35 (d, J = 8.9 Hz, 1H), 3.07 (t, J = 35.5 Hz, 2H), 1.44 (s, 2H), 1.26 (s, 2H), 0.79 (dd, J = 7.1, 2.8 Hz, 3H). | 401.0 |
| 1-250 | $^1$H NMR (400 MHz, $CDCl_3$) δ 8.07 (dd, J = 6.6, 1.8 Hz, 1H), 8.02-7.94 (m, 2H), 7.88-7.80 (m, 1H), 7.55-7.44 (m, 2H), 7.34 (t, J = 9.2 Hz, 1H), 3.14-2.76 (m, 3H). | 359.28 |
| 1-251 | $^I$HNMR (400 MHz, $CDCl_3$) δ 8.17 (s, 2H), 8.08 (s, 1H), 8.01-7.94 (m, 1H), 7.93-7.86 (m, 1H), 7.64-7.52 (m, 2H). | 463.0 |
| 1-252 | $^1$H NMR (400 MHz, $CDCl_3$) δ 8.26 (s, 2H), 8.05 (s, 1H), 8.03-7.99 (m, 1H), 7.91-7.85 (m, 1H), 7.56-7.50 (m, 2H), 3.05 (s, 3H). | 409.27 |
| 1-253 | $^1$H NMR (400 MHz, $CDCl_3$) δ 8.06 (qd, J = 7.7, 3.1 Hz, 2H), 8.01-7.94 (m, 1H), 7.88-7.80 (m, 1H), 7.56-7.42 (m, 2H), 7.33 (t, J = 9.3 Hz, 1H), 2.71-2.10 (m, 1H), 1.21-1.05 (m, 2H), 1.04-0.74 (m, 2H). | 385.36 |
| 1-254 | $^I$HNMR (400 MHz, $CDCl_3$) δ 8.26 (s, 2H), 8.05 (s, 1H), 8.02-7.97 (m, 1H), 7.92-7.84 (m, 1H), 7.57-7.46 (m, 2H), 3.33-3.04 (m, 2H), 1.65-1.44 (m, 2H), 0.98-0.66 (m, 3H). | 436.0 |
| 1-255 | $^I$HNMR (400 MHz, $CDCl_3$) δ 8.25 (s, 2H), 8.05 (s, 1H), 8.02-7.96 (m, 1H), 7.91-7.84 (m, 1H), 7.56-7.45 (m, 2H), 3.30-2.98 (m, 2H), 1.49-1.34 (m, 2H), 1.33-1.15 (m, 2H), 0.87-0.65 (m, 3H). | 451.13 |
| 1-256 | $^1$H NMR (400 MHz, $CDCl_3$) δ 8.27 (s, 2H), 8.06 (d, J = 9.2 Hz, 2H), 7.92-7.80 (m, 1H), 7.57-7.43 (m, 2H), 2.67-2.46 (m, 1H), 1.09 (t, J = 9.1 Hz, 2H), 1.03-0.86 (m, 2H). | 435.15 |
| 1-257 | $^1$H NMR (400 MHz, $CDCl_3$) δ 8.24 (s, 2H), 8.07 (s, 1H), 7.88 (dd, J = 9.9, 7.0 Hz, 1H), 7.66 (dd, J = 9.4, 7.2 Hz, 1H), 3.47-2.97 (m, 2H), 1.28-0.79 (m, 3H). | 459.24 |
| 1-258 | $^1$H NMR (400 MHz, $CDCl_3$) δ 8.17 (s, 1H), 8.03-7.95 (m, 1H), 7.86-7.80 (m, 1H), 7.79-7.73 (m, 1H), 7.50-7.36 (m, 2H), 7.26-7.19 (m, 1H), 3.28-2.97 (m, 2H), 1.08 (t, J = 7.4 Hz, 3H). | 369.4, 371.47 |

(continued)

| Compound No. | $^1$H NMR (400 MHz, $CDCl_3$) | LC-MS ($M+H^+$) |
|---|---|---|
| 1-259 | $^1$H NMR (400 MHz, $CDCl_3$) δ 8.25 (s, 2H), 8.04 (s, 1H), 7.91 (s, 1H), 7.90-7.85 (m, 1H), 7.58 (dd, J = 8.7, 1.8 Hz, 1H), 3.31-3.02 (m, 2H), 1.40 (d, J= 17.2 Hz, 9H), 1.11-0.93 (m, 3H). | 479.14 |
| 1-260 | $^1$H NMR (400 MHz, $CDCl_3$) δ 8.25 (s, 2H), 8.09 (d, J = 12.1 Hz, 1H), 8.07 (s, 1H), 7.73 (dd, J = 15.7, 2.0 Hz, 1H), 7.45-7.35 (m, 1H), 3.33-3.04 (m, 2H), 1.12 (t, J = 7.4 Hz, 3H). | 507.06 |
| 1-261 | $^1$H NMR (400 MHz, $CDCl_3$) 7.97 (dd, J = 9.0, 4.5 Hz, 1H), 7.82 (dd, J = 8.9, 4.8 Hz, 2H), 7.75 (d, J = 2.4 Hz, 1H), 7.73 (d, J = 2.4 Hz, 1H), 7.55 | 444.1 |
| | (dd, J = 8.3, 2.4 Hz, 1H), 3.68-2.87 (m, 2H), 1.63-0.71 (m, 3H). | |
| 1-262 | $^l$HNMR (400 MHz, $CDCl_3$) δ 8.22 (d, J = 12.9 Hz, 2H), 8.02 (d, J = 12.5 Hz, 1H), 7.79 (dd, J = 12.9, 8.2 Hz, 1H), 7.44-7.33 (m, 1H), 7.33 (s, 1H), 3.17 (dt, J= 14.7, 7.2 Hz, 2H), 2.55 (dd, J= 103.7, 16.6 Hz, 3H), 1.41-0.57 (m, 3H). | 437.26 |
| 1-263 | $^l$HNMR (400 MHz, $CDCl_3$) δ 8.25 (s, 2H), 8.02 (s, 1H), 7.75 (s, 1H), 7.61 (s, 1H), 3.28-2.93 (m, 2H), 2.45 (s, 3H), 2.42 (s, 3H), 1.09-0.93 (m, 3H) | 451.37 |
| 1-264 | $^1$H NMR (400 MHz, $CDCl_3$) 8.04 (s, 1H), 7.86 (d, J = 8.4 Hz, 1H), 7.78 (d, J = 10.7 Hz, 1H), 7.72 (t, J = 8.1 Hz, 1H), 7.63 (d, J = 13.9 Hz, 1H), 7.32 (d, J = 8.2 Hz, 1H), 3.29-3.03 (m, 2H), 2.72-2.46 (m, 3H), 1.20-0.83 (m, 3H). | 436.0 |
| 1-265 | $^1$H NMR (400 MHz, $CDCl_3$) δ 8.24 (s, 2H), 8.16 (d, J= 10.7 Hz, 1H), 8.07 (s, 1H), 7.95 (d, J = 13.5 Hz, 1H), 3.20 (q, J = 7.4 Hz, 2H), 1.23-0.82 (m, 3H). | 490.78 |
| 1-266 | $^1$H NMR (400 MHz, $CDCl_3$) δ 8.26 (s, 2H), 8.18-8.13 (m, 1H), 8.13 (s, 1H), 8.09 (s, 1H), 7.77 (d, J = 8.7 Hz, 1H), 3.49-2.91 (m, 2H), 1.04 (dt, J = 12.0, 6.6 Hz, 3H). | 491.33 |
| 1-267 | $^1$H NMR (400 MHz, $CDCl_3$) δ 8.05-7.96 (m, 1H), 7.87-7.79 (m, 1H), 7.48-7.40 (m, 2H), 7.20-7.10 (m, 1H), 3.22-3.02 (m, 2H), 2.37 (d, J = 15.8 Hz, 6H), 1.19-0.91 (m, 3H). | 315.36 |

In the present disclosure, the salt in the benzimidazole compound or the salt thereof includes, but is not limited to, inorganic salts such as hydrochloride, sulfate, nitrate, and phosphate; and organic salts such as acetate, fumarate, maleate, oxalate, methanesulfonate, benzenesulfonate, and p-toluenesulfonate. As previously described, a second aspect of the present disclosure provides a method for preparing a benzimidazole compound, including:

(1) subjecting a compound V and a compound IV to a first reaction in a first solvent in the presence of a first alkaline substance and a condensing agent to obtain a compound III;

(2) subjecting the compound III and an acidic substance to a second reaction in a second solvent to obtain a compound II; and

(3) subjecting the compound II and a sulfonyl-containing compound to a third reaction in a third solvent in the presence of a second alkaline substance to obtain the benzimidazole compound;

wherein the compound V has a structure represented by a formula (V), the compound IV has a structure represented by a formula (IV), the compound III has a structure represented by a formula (III), the compound II has a structure represented by a formula (II), the benzimidazole compound has a structure represented by a formula (I), and the sulfonyl-containing compound has a structure represented by a formula (VI);

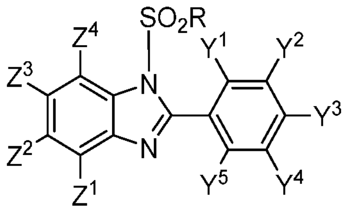 Formula (I); 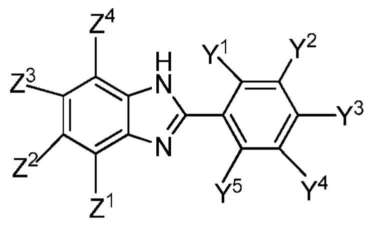 Formula (II);

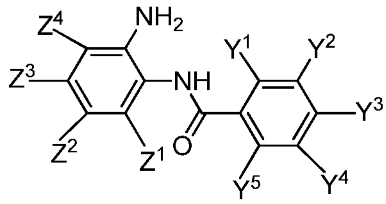 Formula (III); 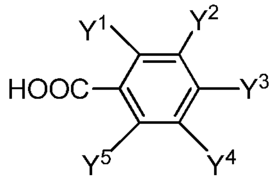 Formula (IV);

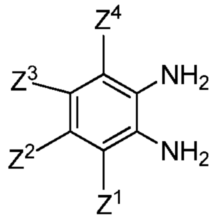 Formula (V); 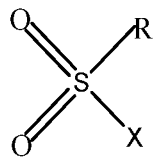 Formula (VI);

in the formula (I), the formula (II), the formula (III), the formula (IV), the formula (V) and the formula (VI), definitions of R, $Y^1$, $Y^2$, $Y^3$, $Y^4$, $Y^5$, $Z^1$, $Z^2$, $Z^3$, and $Z^4$ are correspondingly the same as those in the first aspect, and X is selected from halogen.

According to the present disclosure, in the step (1), the first reaction is shown below:

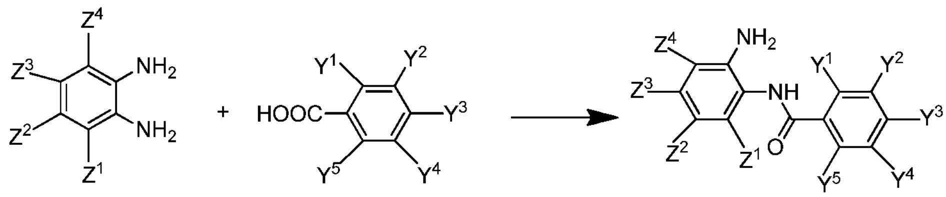

V IV III

preferably, in the step (1), the conditions of the first reaction include a temperature of -10°C to 150°C and a reaction time of 0.5-48 h.

Preferably, in the step (1), the compound V and the compound IV are used in a molar ratio of 0.5-2:1. The compound V and the compound IV are commercially available.

Preferably, in the step (1), the condensing agent is selected from at least one of 1-ethyl-3-(3-dimethylaminopropyl)carbodiimide (EDC) or a hydrochloride thereof (EDCI), carbonyldiimidazole (CDI), 1,3-dicyclohexylcarbodiimide (DCC), diethyl cyanophosphate (DEPC), chlorocarbonates, and 2-chloro-1-methylpyridinium iodide.

Preferably, in the step (1), the condensing agent and the compound IV are used in a molar ratio of 1-2:1.

Preferably, in the step (1), the first alkaline substance is selected from at least one of pyridine, dimethylaminopyridine (DMAP), triethylamine, diisopropylethylamine, sodium hydroxide, potassium hydroxide, sodium carbonate, potassium carbonate, sodium bicarbonate, potassium bicarbonate, sodium acetate, potassium acetate, sodium methoxide, sodium ethoxide, sodium tert-butoxide, potassium tert-butoxide, and 1,8-diazabicyclo[5.4.0]undec-7-ene.

Preferably, in the step (1), the first alkaline substance and the compound IV are used in a molar ratio of 0.1-10:1.

Preferably, in the step (1), the first solvent is selected from at least one of pyridine, dichloromethane, chloroform, carbon tetrachloride, benzene, toluene, xylene, chlorobenzene, dichlorobenzene, diethyl ether, methyl tert-butyl ether, dioxane, tetrahydrofuran, 2-methyltetrahydrofuran, N,N-dimethylformamide (DMF), N,N-dimethylacetamide (DMA), N-methylpyrrolidone (NMP), acetone, methyl ethyl ketone, dimethyl sulfoxide, and 1,3-dimethyl-2-imidazolidinone.

Preferably, in the step (1), the first solvent is used in an amount of 1-20 mL relative to 1 mmol of the compound IV.

According to the present disclosure, in the step (1), the first solvent and the first alkaline substance may be the same, and may simultaneously be, for example, pyridine. Moreover, it should be specifically noted that when the first solvent and the first alkaline substance are the same substance, both need to be measured separately.

According to the present disclosure, in the step (2), the second reaction is shown below:

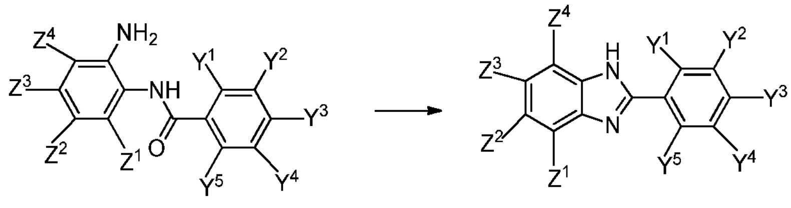

III II

preferably, in the step (2), the conditions of the second reaction include a temperature of -10°C to 300°C, and a reaction time of 0.5-48 h.

Preferably, in the step (2), the acidic substance is selected from at least one of p-toluenesulfonic acid or a hydrate thereof, methanesulfonic acid, trifluoromethanesulfonic acid, hydrochloric acid, sulfuric acid, nitric acid, formic acid, acetic acid, propionic acid, trifluoroacetic acid, trichloroacetic acid, benzoic acid, and phosphoric acid. The p-toluenesulfonic acid hydrate is preferably p-toluenesulfonic acid monohydrate.

Preferably, in the step (2), the acidic substance and the compound IV are used in a molar ratio of 0.01-10:1.

Preferably, in the step (2), the second solvent is selected from at least one of N,N-dimethylformamide (DMF), N,N-dimethylacetamide (DMA), N-methylpyrrolidone (NMP), diethyl ether, methyl tert-butyl ether, dioxane, tetrahydrofuran, 2-methyltetrahydrofuran, benzene, toluene, xylene, acetone, methyl ethyl ketone, dichloromethane, chloroform, carbon tetrachloride, chlorobenzene, dichlorobenzene, ethyl acetate, dimethyl sulfoxide, and 1,3-dimethyl-2-imidazolidinone.

Preferably, in the step (2), the second solvent is used in an amount of 1-20 mL relative to 1 mmol of the compound III.

Preferably, the step (2) further includes adjusting a pH of the system to 7-10 by using an alkaline solution after the second reaction, wherein the alkaline solution is, for example, an aqueous sodium hydroxide solution.

According to the present disclosure, in the step (3), the third reaction is shown below:

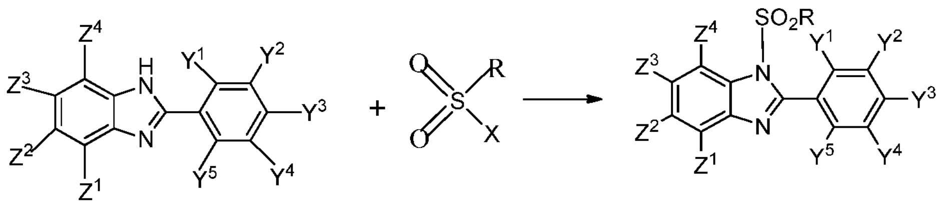

II VI I

preferably, in the step (3), the conditions of the third reaction include a temperature of -10°C to 100°C, and a reaction time of 0.5-48 h.

Preferably, in the step (3), the sulfonyl-containing compound and the compound II are used in a molar ratio of 0.8-10:1.

Preferably, in the step (3), the sulfonyl-containing compound is selected from at least one of methylsulfonyl chloride, ethylsulfonyl chloride, n-propylsulfonyl chloride and n-butylsulfonyl chloride, more preferably ethylsulfonyl chloride.

Preferably, in the step (3), the second alkaline substance is selected from at least one of pyridine, dimethylaminopyridine (DMAP), triethylamine, diisopropylethylamine, sodium hydride, potassium hydride, sodium hydroxide, potassium hydroxide, sodium carbonate, potassium carbonate, sodium bicarbonate, potassium bicarbonate, sodium acetate, potassium acetate, sodium methoxide, sodium ethoxide, sodium tert-butoxide, potassium tert-butoxide, and 1,8-diazabicyclo[5.4.0]undec-7-ene. Preferably, in the step (3), the second alkaline substance and the compound II are used in a molar ratio of 1-10:1.

Preferably, in the step (3), the third solvent is selected from at least one of diethyl ether, tetrahydrofuran (THF), 2-methyltetrahydrofuran, N,N-dimethylformamide (DMF), N,N-dimethylacetamide (DMA), N-methylpyrrolidone (NMP), dioxane, benzene, toluene, xylene, dichloromethane, chloroform, carbon tetrachloride, chlorobenzene, and dichlorobenzene.

Preferably, in the step (3), the third solvent is used in an amount of 1-20 mL relative to 1 mmol of the compound II.

According to one preferred specific embodiment of the present disclosure, the step (3) includes subjecting the compound II to a third reaction in a first stage in a third solvent in the presence of a second alkaline substance, then adding the sulfonyl-containing compound, and carrying out a third reaction in a second stage to obtain the benzimidazole compound.

Preferably, the conditions of the third reaction in the first stage include: a temperature of -10°C to 100°C, and a reaction time of 10-120 min.

Preferably, the conditions of the third reaction in the second stage include: a temperature of -10°C to 100°C, and a reaction time of 0.5-48 h.

According to the present disclosure, various aftertreatment operations such as extraction, washing, drying, suction filtration, concentration, separation and purification and the like, which are currently used in the art, can be further included in the method, which are not particularly limited in the present disclosure, and can be performed by various conventional operations in the art, for example, the extraction is performed with ethyl acetate; the drying is performed by using anhydrous sodium sulfate; the concentration is performed under reduced pressure; the separation and purification are performed by column chromatography, and the like.

As previously described, a third aspect of the present disclosure provides a benzimidazole compound prepared by the method described in the second aspect.

As previously described, a fourth aspect of the present disclosure provides use of the benzimidazole compound or the salt thereof according to the first aspect or the third aspect for the preparation of an insecticide and acaricide.

Preferably, the benzimidazole compound or the salt thereof is used as an active ingredient (i.e., an effective ingredient) in the insecticide and acaricide.

As previously described, a fifth aspect of the present disclosure provides an insecticide and acaricide, including an active ingredient selected from at least one of the benzimidazole compound or the salt thereof according to the first aspect or the third aspect.

In the fifth aspect of the present disclosure, preferably, based on the total weight of the insecticide and acaricide, the content of the active ingredient ranges from 1 wt% to 99 wt%, for example, the content of the active ingredient is 1 wt%, 5 wt%, 10 wt%, 15 wt%, 20 wt%, 25 wt%, 30 wt%, 35 wt%, 40 wt%, 45 wt%, 50 wt%, 55 wt%, 60 wt%, 65 wt%, 70 wt%, 75 wt%, 80 wt%, 85 wt%, 90 wt%, 95 wt%, 99 wt% or any value in a range consisting of any two of these point values. More preferably, the content of the active ingredient ranges from 5 wt% to 60 wt% based on the total weight of the insecticide and acaricide.

In the fifth aspect of the present disclosure, preferably, the insecticide and acaricide further includes a carrier. Preferably, the carrier in the insecticide and acaricide is a substance which is agriculturally, forestally and horticulturally acceptable and facilitates application of the active ingredient. Particularly preferably, the carrier is a liquid carrier and/or a solid carrier, wherein the solid carrier is preferably at least one solid substance selected from syderolite, natural or synthetic silicate, silica, resin, wax, and a solid fertilizer; and the liquid carrier is preferably a liquid substance selected from water, alcohols, ketones, a petroleum fraction, aromatic hydrocarbon, chlorinated hydrocarbon, and liquefied gas.

In the fifth aspect of the present disclosure, the insecticide and acaricide may also contain other auxiliary components commonly used in the art, such as a surfactant, a protective colloid, a binder, a thickener, a thixotropic agent, a penetrant, a chelating agent, a colorant, and a polymer, which is not particularly limited in the present disclosure, and those skilled in the art can select the reasonable composition and dosage according to actual needs.

In the fifth aspect of the present disclosure, preferably, dosage forms of the insecticide and acaricide are each independently selected from at least one of wettable powder, soluble powder, an emulsifiable concentrate, an aqueous suspension, a dispersible oil suspension, an aqueous emulsion, a suspoemulsion, a microemulsion, an aqueous solution, granules, microcapsules and water dispersible granules, therefore, the active ingredient is more easily dissolved or dispersed so as to be more easily dispersed when used as an active substance of the insecticide and acaricide, improving the application effect.

In the fifth aspect of the present disclosure, a method for preparation of the insecticide and acaricide is not particularly limited in the present disclosure, and those skilled in the art can refer to methods in the literatures and standards existing in the art or use methods existing in the art to prepare a reagent of the desired composition and dosage form.

As previously described, a sixth aspect of the present disclosure provides use of the insecticide and acaricide for killing pests and/or mites in agriculture, forestry and horticulture.

Preferably, a manner of the use includes applying the insecticide and acaricide to pests and/or pest mites, or applying the insecticide and acaricide to a growth medium of the pests and/or pest mites. According to the present disclosure, the growth medium is, for example, a plant or soil.

Preferably, the effective amount applied (i.e., the usage amount of the active ingredient) is in the range from 10 g to 1000 g per hectare of soil, more preferably from 20 g to 500 g per hectare of soil. According to the present

disclosure, a specific mode of application is not particularly limited, and can be carried out by using methods conventional in the art, such as spraying the insecticide and acaricide onto the pests and/or pest mites, or spraying the insecticide and acaricide onto the growth medium of the pests and/or pest mites.

According to the present disclosure, in the use of the insecticide and acaricide for killing pests and mites in agriculture, forestry and horticulture, the insecticide and acaricide can also be applied in combination with substances such as a bactericide, an insecticide, a herbicide, a plant growth regulator, a plant fertilizer and the like existing in the art, whereby the combination produces additive or synergistic effects, thereby obtaining better results. The present disclosure is not particularly limited thereto, and those skilled in the art can reasonably select a suitable amount of substances according to actual requirements for combination and compounding use on the premise of using the benzimidazole compound or the salt thereof of the present disclosure as an active ingredient.

Hereinafter, the present disclosure will be described in detail by examples.

In the following examples, unless otherwise specified, the raw materials used are commercially available.

Sources of some of the raw materials are shown in Table 3:

Table 3

| Raw material | Purchased from manufacturer |
|---|---|
| V-1 | Beijing Ouhe Technology Co., Ltd. |
| V-2 | Bidepharm |
| IV-1 | Beijing Ouhe Technology Co., Ltd. |
| IV-2 | Beijing Ouhe Technology Co., Ltd. |
| IV-3 | Beijing Ouhe Technology Co., Ltd. |
| IV-4 | Beijing Ouhe Technology Co., Ltd. |
| Condensing agent: EDCI | Beijing Ouhe Technology Co., Ltd. |
| First alkaline substance: DMAP | Beijing Ouhe Technology Co., Ltd. |
| Acidic substance: p-toluenesulfonic acid monohydrate | Beijing Ouhe Technology Co., Ltd. |
| Sulfonyl-containing compound: ethylsulfonyl chloride | Beijing Ouhe Technology Co., Ltd. |

In the following examples, under otherwise specified, room temperature refers to 25±2°C.

Example 1

This example is preparation of a compound 1-27

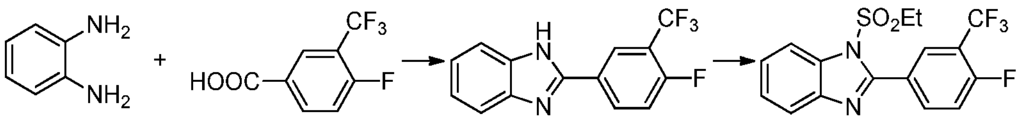

V-1 IV-1 II-1 1-27

A specific preparation process was as follows:

(1) a first solvent (pyridine, 15 mL) was added in a reaction flask, a condensing agent (EDCI, 15 mmol) and a first alkaline substance (DMAP, 2 mmol) were added, a compound V (V-1, 10 mmol) and a compound IV (IV-1, 10 mmol) were added, a first reaction was carried out at room temperature for 3 h, then water was added, extraction was performed with ethyl acetate, and an organic phase was washed with saturated brine, and dried over anhydrous sodium sulfate. Suction filtration was performed, and the obtained filtrate was concentrated under reduced pressure to give a compound III;

(2) the obtained compound III was dissolved in a second solvent (NMP, 15 mL), an acidic substance (p-toluenesulfonic acid monohydrate, 30 mmol) was added, and a second reaction was carried out at 160°C for 4 h. The resulting reaction solution was cooled to room temperature, a 10 wt% aqueous sodium hydroxide solution was added to adjust a pH value of the system to be 9, then extraction was performed with ethyl acetate, an organic phase was washed with saturated brine, and dried over anhydrous sodium sulfate, suction filtration was performed, and the

obtained filtrate was concentrated under reduced pressure to give a compound II (II-1, 8 mmol); and

(3) the compound II was dissolved in a third solvent (THF, 30 mL), a second alkaline substance (0.96 g of NaH wrapped in mineral oil, the NaH content is 60 wt%, and NaH is 24 mmol) was added, a reaction was carried out at room temperature for 10 min, a sulfonyl-containing compound (ethylsulfonyl chloride, 24 mmol) was added, and a reaction was carried out at room temperature for 12 h. Saturated ammonium chloride was added for quenching, extraction was performed with ethyl acetate, an organic phase was washed with saturated brine, and dried over anhydrous sodium sulfate, suction filtration was performed, the obtained filtrate was concentrated under reduced pressure, and the obtained product was separated and purified by column chromatography to give the compound 1-27 (2.08 g).

Example 2

This example is preparation of a compound 1-28

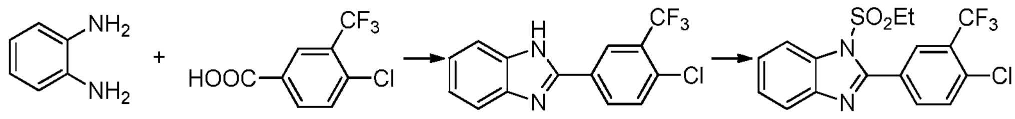

V-1 IV-2 II-2 1-28

A specific preparation process was as follows:

(1) a first solvent (pyridine, 15 mL) was added in a reaction flask, a condensing agent (EDCI, 15 mmol) and a first alkaline substance (DMAP, 2 mmol) were added, a compound V (V-1, 10 mmol) and a compound IV (IV-2, 10 mmol) were added, a first reaction was carried out at room temperature for 3 h, then water was added, extraction was performed with ethyl acetate, an organic phase was washed with saturated brine, and dried over anhydrous sodium sulfate, suction filtration was performed, and the obtained filtrate was concentrated under reduced pressure to give a compound III;

(2) the obtained compound III was dissolved in a second solvent (NMP, 15 mL), an acidic substance (p-toluenesulfonic acid monohydrate, 30 mmol) was added, and a second reaction was carried out at 160°C for 4 h. The resulting reaction solution was cooled to room temperature, a 10 wt% aqueous sodium hydroxide solution was added to adjust a pH value of the system to be 10, then extraction was performed with ethyl acetate, an organic phase was washed with saturated brine, and dried over anhydrous sodium sulfate, suction filtration was performed, and the obtained filtrate was concentrated under reduced pressure to give a compound II (II-2, 8.2 mmol); and

(3) the compound II was dissolved in a third solvent (THF, 30 mL), a second alkaline substance (0.98 g of NaH wrapped in mineral oil, the NaH content is 60 wt%, and NaH is 24.6 mmol) was added, a reaction was carried out at room temperature for 10 min, a sulfonyl-containing compound (ethylsulfonyl chloride, 24.6 mmol) was added, and a reaction was carried out at room temperature for 12 h. After the reaction, saturated ammonium chloride was added for quenching, extraction was performed with ethyl acetate, an organic phase was washed with saturated brine, and dried over anhydrous sodium sulfate, suction filtration was performed, the obtained filtrate was concentrated under reduced pressure, and the obtained product was separated and purified by column chromatography to give the compound 1-28 (2.10 g).

Example 3

This example is preparation of a compound 1-47

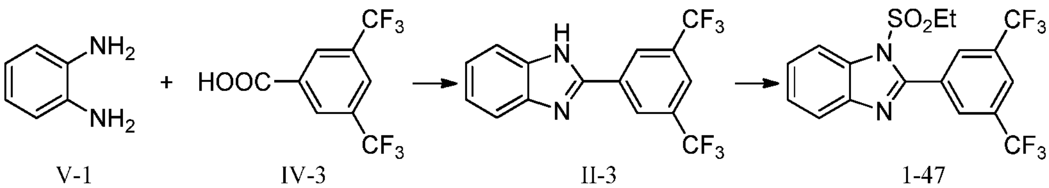

V-1 IV-3 II-3 1-47

A specific preparation process was as follows:

(1) a first solvent (pyridine, 15 mL) was added in a reaction flask, a condensing agent (EDCI, 15 mmol) and a first alkaline substance (DMAP, 2 mmol) were added, a compound V (Formula V-1, 10 mmol) and a compound IV (IV-3, 10 mmol) were added, a first reaction was carried out at room temperature for 3 h, then water was added, extraction was performed with ethyl acetate, an organic phase was washed with saturated brine, and dried over anhydrous sodium sulfate, suction filtration was performed, and the obtained filtrate was concentrated under reduced pressure to give a compound III;

(2) the obtained compound III was dissolved in a second solvent (NMP, 15 mL), an acidic substance (p-toluenesulfonic acid monohydrate, 30 mmol) was added, and a second reaction was carried out at 160°C for 4 h. The resulting reaction solution was cooled to room temperature, a 10 wt% aqueous sodium hydroxide solution was added to adjust a pH value of the system to be 10, then extraction was performed with ethyl acetate, an organic phase was washed with saturated brine, and dried over anhydrous sodium sulfate, suction filtration was performed, and the obtained filtrate was concentrated under reduced pressure to give a compound II (II-3, 8 mmol); and

(3) the compound II was dissolved in a third solvent (THF, 30 mL), a second alkaline substance (0.96 g of NaH wrapped in mineral oil, the NaH content is 60 wt%, and NaH is 24 mmol) was added, a reaction was carried out at room temperature for 10 min, a sulfonyl-containing compound (ethylsulfonyl chloride, 24 mmol) was added, and a reaction was carried out at room temperature for 12 h. After the reaction, saturated ammonium chloride was added for quenching, extraction was performed with ethyl acetate, and an organic phase was washed with saturated brine, and dried over anhydrous sodium sulfate. Suction filtration was performed, the obtained filtrate was concentrated under reduced pressure, and the obtained product was separated and purified by column chromatography to give the compound 1-47 (2.53 g).

Example 4

This example is preparation of a compound 1-56

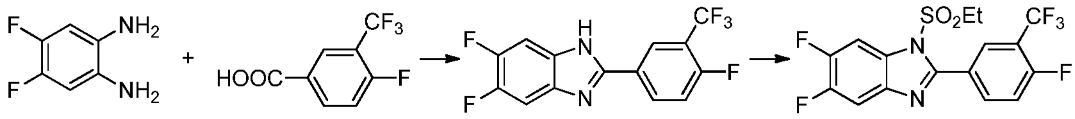

V-2 IV-1 II-4 1-56

A specific preparation process was as follows:

(1) a first solvent (pyridine, 15 mL) was added in a reaction flask, a condensing agent (EDCI, 15 mmol) and a first alkaline substance (DMAP, 2 mmol) were added, a compound V (V-2, 10 mmol) and a compound IV (IV-1, 10 mmol) were added, a first reaction was carried out at room temperature for 3 h, then water was added, extraction was performed with ethyl acetate, an organic phase was washed with saturated brine, and dried over anhydrous sodium sulfate, suction filtration was performed, and the obtained filtrate was concentrated under reduced pressure to give a compound III;

(2) the obtained compound III was dissolved in a second solvent (NMP, 15 mL), an acidic substance (p-toluenesulfonic acid monohydrate, 30 mmol) was added, and a second reaction was carried out at 160°C for 4 h. The resulting reaction solution was cooled to room temperature, a 10 wt% aqueous sodium hydroxide solution was added to adjust a pH value of the system to be 10, then extraction was performed with ethyl acetate, an organic phase was washed with saturated brine, and dried over anhydrous sodium sulfate, suction filtration was performed, and the obtained filtrate was concentrated under reduced pressure to give a compound II (II-4, 7.5 mmol); and

(3) the compound II was dissolved in a third solvent (THF, 30 mL), a second alkaline substance (0.90 g of NaH wrapped in mineral oil, the NaH content is 60 wt%, and NaH is 22.5 mmol) was added, a reaction was carried out at room temperature for 10 min, a sulfonyl-containing compound (ethylsulfonyl chloride, 22.5 mmol) was added, and a reaction was continued to be carried out at room temperature for 12 h. After the reaction, saturated ammonium chloride was added for quenching, extraction was performed with ethyl acetate, an organic phase was washed with saturated brine, and dried over anhydrous sodium sulfate, suction filtration was performed, the obtained filtrate was concentrated under reduced pressure, and the obtained product was separated and purified by column chromatography to give the compound 1-56 (2.16 g).

Example 5

This example is preparation of a compound 1-57

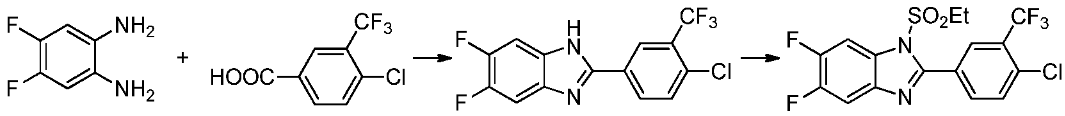

V-2 IV-2 II-5 1-57

A specific preparation process was as follows:

(1) a first solvent (15 mL of pyridine) was added in a reaction flask, a condensing agent (EDCI, 15 mmol) and a first alkaline substance (DMAP, 2 mmol) were added, a compound V (V-2, 10 mmol) and a compound IV (IV-2, 10 mmol) were added, a first reaction was carried out at room temperature for 3 h, water was added, extraction was performed with ethyl acetate, and an organic phase was washed with saturated brine, and dried over anhydrous sodium sulfate. Suction filtration was performed, and the obtained filtrate was concentrated under reduced pressure to give a compound III;
(2) the obtained compound III was dissolved in a second solvent (NMP, 15 mL), an acidic substance (p-toluenesulfonic acid monohydrate, 30 mmol) was added, and a second reaction was carried out at 160°C for 4 h. The resulting reaction solution was cooled to room temperature, a 10 wt% aqueous sodium hydroxide solution was added to adjust a pH value of the system to be 9, then extraction was performed with ethyl acetate, an organic phase was washed with saturated brine, and dried over anhydrous sodium sulfate, suction filtration was performed, and the obtained filtrate was concentrated under reduced pressure to give a compound II (II-5, 7.23 mmol); and
(3) the compound II was dissolved in a third solvent (THF, 30 mL), a second alkaline substance (0.86 g of NaH wrapped in mineral oil, the NaH content is 60 wt%, and NaH is 21.6 mmol) was added, a reaction was carried out at room temperature for 10 min, a sulfonyl-containing compound (ethylsulfonyl chloride, 21.6 mmol) was added, and a reaction was continued to be carried out at room temperature for 12 h. After the reaction, saturated ammonium chloride was added for quenching, extraction was performed with ethyl acetate, and an organic phase was washed with saturated brine, and dried over anhydrous sodium sulfate. Suction filtration was performed, the obtained filtrate was concentrated under reduced pressure, and the obtained product was separated and purified by column chromatography to give the compound 1-57 (2.12 g).

Example 6

This example is preparation of a compound 1-213

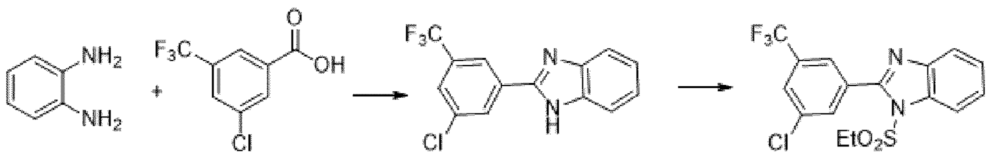

V-1 IV-4 II-6 1-213

A specific preparation process was as follows:

(1) a first solvent (pyridine, 15 mL) was added in a reaction flask, a condensing agent (EDCI, 15 mmol) and a first alkaline substance (DMAP, 2 mmol) were added, a compound V (V-1, 10 mmol) and a compound IV (IV-4, 10 mmol) were added, a first reaction was carried out at room temperature for 3 h, then water was added, extraction was performed with ethyl acetate, an organic phase was washed with saturated brine, and dried over anhydrous sodium sulfate, suction filtration was performed, and the obtained filtrate was concentrated under reduced pressure to give a compound III;
(2) the obtained compound III was dissolved in a second solvent (NMP, 15 mL), an acidic substance (p-toluenesulfonic acid monohydrate, 30 mmol) was added, and a second reaction was carried out at 160°C for 4 h. The resulting reaction solution was cooled to room temperature, a 10 wt% aqueous sodium hydroxide solution was added to adjust a pH value of the system to be 10, then extraction was performed with ethyl acetate, an organic phase was washed with saturated brine, and dried over anhydrous sodium sulfate, suction filtration was performed, and the obtained filtrate was concentrated under reduced pressure to give a compound II (II-6, 8.2 mmol); and
(3) the compound II was dissolved in a third solvent (THF, 30 mL), a second alkaline substance (0.98 g of NaH wrapped in mineral oil, the NaH content is 60 wt%, and NaH is 24.6 mmol) was added, a reaction was carried out at room temperature for 10 min, a sulfonyl-containing compound (ethylsulfonyl chloride, 24.6 mmol) was added,

and a reaction was carried out at room temperature for 12 h. After the reaction, saturated ammonium chloride was added for quenching, extraction was performed with ethyl acetate, an organic phase was washed with saturated brine, and dried over anhydrous sodium sulfate, suction filtration was performed, the obtained filtrate was concentrated under reduced pressure, and the obtained product was separated and purified by column chromatography to give the compound 1-213 (2.10 g).

Remaining examples

The following compounds were prepared in a similar manner to that in Example 1, except that the type and/or amount of raw materials were different, and the rest were the same as those in Example 1 to give a compound 1-25, a compound 1-26, a compound 1-29, a compound 1-30, a compound 1-31, a compound 1-32, a compound 1-33, a compound 1-34, a compound 1-37, a compound 1-38, a compound 1-40, a compound 1-43, a compound 1-49, a compound 1-50, a compound 1-51, a compound 1-52, a compound 1-54, a compound 1-55, a compound 1-60, a compound 1-61, a compound 1-62, a compound 1-68, a compound 1-69, a compound 1-72, a compound 1-73, a compound 1-75, a compound 1-76, a compound 1-81, a compound 1-133, a compound 1-174, a compound 1-186, a compound 1-214, a compound 1-218, a compound 1-235, a compound 1-239, a compound 1-254, a compound 1-255, a compound 1-257, a compound 1-261, a compound 1-264, and a compound 1-265, respectively.

Biological activity test

This test example tested the acaricidal activity of the compounds prepared above, in particular against Tetranychus cinnabarinus, and a specific test process was as follows:

(1) compounds to be tested were dissolved with acetone, and the resulting solution was diluted with a 0.1 wt% Tween 80 aqueous solution to a desired concentration (see test examples 1-A to 1-D below in detail) to prepare medicaments, with the acetone content of not more than 5 wt%;
(2) one real leaf was removed from kidney bean seedlings grown to two real leaves, and the number of mites inoculated was investigated 24 hours after inoculation of Tetranychus cinnabarinus (the number of Tetranychus cinnabarinus inoculated for each kidney bean seedling was 50-150). The whole plants of three kidney bean seedlings were separately sprayed with the medicaments prepared in the step (1) by using a hand-held sprayer (the spray amount was 0.5 mL per plant). After the treatment, the kidney bean seedlings were placed in a constant temperature observation room (25°C) for observation, the number of living mites was investigated after 72 hours, and the lethality was calculated:

Lethality (%)=(the number of mites inoculated-the number of living mites after the medicaments are sprayed)/the number of mites inoculated×100.

Test example 1-A

The compounds were diluted to a concentration of 100 mg/L and tested according to the above procedures. In this test, the following compounds showed more than 90% lethality at a concentration of 100 mg/L, and the specific results are shown in Table 4:
the compound 1-25, the compound 1-26, the compound 1-27, the compound 1-28, the compound 1-29, the compound 1-30, the compound 1-31, the compound 1-32, the compound 1-33, the compound 1-34, the compound 1-37, the compound 1-38, the compound 1-40, the compound 1-43, the compound 1-47, the compound 1-49, the compound 1-50, the compound 1-51, the compound 1-52, the compound 1-54, the compound 1-55, the compound 1-56, the compound 1-57, the compound 1-60, the compound 1-61, the compound 1-62, the compound 1-68, the compound 1-69, the compound 1-72, the compound 1-73, the compound 1-75, the compound 1-76, the compound 1-81, the compound 1-133, the compound 1-174, the compound 1-186, the compound 1-213, the compound 1-214, the compound 1-218, the compound 1-235, the compound 1-239, the compound 1-254, the compound 1-255, the compound 1-257, the compound 1-261, the compound 1-264, and the compound 1-265.

Test example 1-B

The compounds were diluted to a concentration of 25 mg/L and tested according to the above procedures. In this test, the following compounds showed more than 90% lethality at a concentration of 25 mg/L, and the specific results

are shown in Table 4:
the compound 1-26, the compound 1-27, the compound 1-28, the compound 1-29, the compound 1-30, the compound 1-31, the compound 1-32, the compound 1-34, the compound 1-37, the compound 1-38, the compound 1-47, the compound 1-49, the compound 1-56, the compound 1-57, the compound 1-61, the compound 1-68, the compound 1-69, the compound 1-73, the compound 1-133, the compound 1-174, the compound 1-186, the compound 1-213, the compound 1-214, the compound 1-218, the compound 1-235, the compound 1-239, the compound 1-254, the compound 1-255, the compound 1-257, the compound 1-261, the compound 1-264, and the compound 1-265.

Test example 1-C

The compounds were diluted to a concentration of 6.25 mg/L and tested according to the above procedures. In this test, the following compounds showed more than 90% lethality at 6.25 mg/L, and the specific results are shown in Table 4:
the compound 1-27, the compound 1-28, the compound 1-47, the compound 1-56, the compound 1-57, the compound 1-133, the compound 1-174, the compound 1-186, the compound 1-213, the compound 1-214, the compound 1-218, the compound 1-235, the compound 1-239, the compound 1-254, the compound 1-255, the compound 1-257, the compound 1-261, the compound 1-264, and the compound 1-265.

Table 4

| Compound type | Lethality/% | | |
|---|---|---|---|
| | 100 mg/L | 25 mg/L | 6.25 mg/L |
| Compound 1-27 | 100 | 100 | 90 |
| Compound 1-28 | 100 | 100 | 97 |
| Compound 1-47 | 100 | 100 | 99 |
| Compound 1-56 | 100 | 100 | 95 |
| Compound 1-57 | 100 | 100 | 92 |
| Compound 1-133 | 100 | 100 | 99 |
| Compound 1-174 | 100 | 98 | 98 |
| Compound 1-186 | 100 | 100 | 100 |
| Compound 1-213 | 100 | 100 | 99 |
| Compound 1-214 | 100 | 100 | 97 |
| Compound 1-218 | 100 | 99 | 98 |
| Compound 1-235 | 100 | 99 | 99 |
| Compound 1-239 | 100 | 100 | 100 |
| Compound 1-254 | 100 | 100 | 97 |
| Compound 1-255 | 100 | 100 | 91 |
| Compound 1-257 | 100 | 100 | 98 |
| Compound 1-261 | 100 | 100 | 99 |
| Compound 1-264 | 100 | 100 | 100 |
| Compound 1-265 | 100 | 100 | 100 |

Comparative test example

The present disclosure also tested the bioassay activity of the following compounds against Tetranychus cinnabarinus, a test process was the same as above, and the test results are shown in Table 5 below:

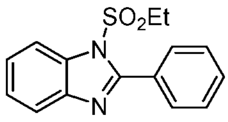

$KC_1$

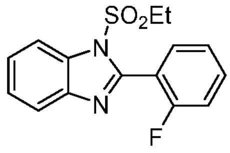

$KC_2$

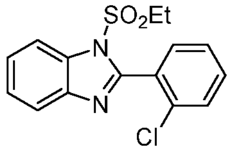

$KC_3$

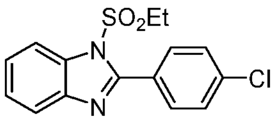

$KC_4$

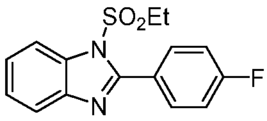

$KC_5$

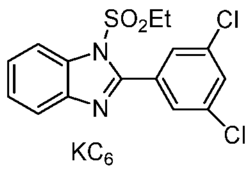

$KC_6$

Table 5

| Compound name | Lethality/% | |
|---|---|---|
| | 100 mg/L | 25 mg/L |
| $KC_1$ | 82 | 52 |
| $KC_2$ | 98 | 49 |
| $KC_3$ | 88 | 70 |
| $KC_4$ | 37 | 57 |
| $KC_5$ | 66 | 40 |
| $KC_6$ | 100 | 86 |

As can be seen from the above test results, the benzimidazole compound or the salt thereof provided by the present disclosure has excellent insecticidal and acaricidal effects, and the insecticidal and acaricidal activity is much higher than that of known compounds, and particularly, the benzimidazole compound of the present disclosure can have excellent insecticidal and acaricidal effects when used at a low concentration (e.g., 6.25 mg/L).

Preferred embodiments of the present disclosure are described above in detail, but the present disclosure is not limited thereto. Within the technical concept range of the present disclosure, the technical solution of the present disclosure can be subjected to various simple variations, including the combinations of various technical features in any other suitable manner, and these simple variations and combinations should likewise be considered as the contents disclosed by the present disclosure, and all fall within the protection scope of the present disclosure.

## Claims

1. A benzimidazole compound or a salt thereof, wherein the benzimidazole compound has a structure represented by a formula (I):

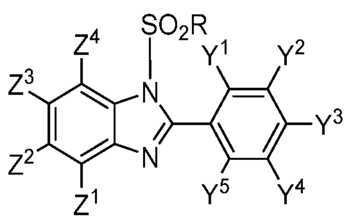 Formula (I);

wherein in the formula (I),

R is selected from substituted or unsubstituted $C_2$-$C_{10}$ alkyl, substituted or unsubstituted $C_3$-$C_{10}$ cycloalkyl, substituted or unsubstituted $C_2$-$C_{10}$ alkenyl, and substituted or unsubstituted $C_2$-$C_{10}$ alkynyl, and optionally

present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy;

$Y^1$, $Y^2$, $Y^3$, $Y^4$, and $Y^5$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3- to 8-membered ring;

$Z^1$, $Z^2$, $Z^3$, and $Z^4$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

**2.** The compound or the salt thereof according to claim 1, wherein in the formula (I),

R is selected from $C_2$-$C_{10}$ alkyl, halo $C_2$-$C_{10}$ alkyl, $C_3$-$C_{10}$ cycloalkyl substituted $C_2$-$C_{10}$ alkyl, halo $C_3$-$C_{10}$ cycloalkyl substituted $C_2$-$C_{10}$ alkyl, $C_3$-$C_{10}$ cycloalkyl substituted halo $C_2$-$C_{10}$ alkyl, $C_1$-$C_{10}$ alkoxy substituted $C_2$-$C_{10}$ alkyl, halo $C_1$-$C_{10}$ alkoxy substituted $C_2$-$C_{10}$ alkyl, $C_1$-$C_{10}$ alkoxy substituted halo $C_2$-$C_{10}$ alkyl, $C_3$-$C_{10}$ cycloalkoxy substituted $C_2$-$C_{10}$ alkyl, halo $C_3$-$C_{10}$ cycloalkoxy substituted $C_2$-$C_{10}$ alkyl, $C_3$-$C_{10}$ cycloalkoxy substituted halo $C_2$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy substituted $C_3$-$C_{10}$ cycloalkyl, halo $C_1$-$C_{10}$ alkoxy substituted $C_3$-$C_{10}$ cycloalkyl, $C_1$-$C_{10}$ alkoxy substituted halo $C_3$-$C_{10}$ cycloalkyl, $C_3$-$C_{10}$ cycloalkoxy substituted $C_3$-$C_{10}$ cycloalkyl, halo $C_3$-$C_{10}$ cycloalkoxy substituted $C_3$-$C_{10}$ cycloalkyl, $C_3$-$C_{10}$ cycloalkoxy substituted halo $C_3$-$C_{10}$ cycloalkyl, $C_2$-$C_{10}$ alkenyl, halo $C_2$-$C_{10}$ alkenyl, $C_2$-$C_{10}$ alkynyl, and halo $C_2$-$C_{10}$ alkynyl;

$Y^1$, $Y^2$, $Y^3$, $Y^4$, and $Y^5$ are each independently selected from H, halogen, CN, $NO_2$, $C_1$-$C_{10}$ alkyl, halo $C_1$-$C_{10}$

alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy, halo $C_1$-$C_{10}$ alkoxy, $C_1$-$C_{10}$ alkylthio, halo $C_1$-$C_{10}$ alkylthio, $C_1$-$C_{10}$ alkylsulfinyl, halo $C_1$-$C_{10}$ alkylsulfinyl, $C_1$-$C_{10}$ alkylsulfonyl, halo $C_1$-$C_{10}$ alkylsulfonyl, formyl, $C_1$-$C_{10}$ alkylcarbonyl, halo $C_1$-$C_{10}$ alkylcarbonyl, $C_1$-$C_{10}$ alkoxycarbonyl, halo $C_1$-$C_{10}$ alkoxycarbonyl, arylcarbonyl, aryloxycarbonyl, $C_2$-$C_6$ alkenyl, $C_2$-$C_6$ alkynyl, halo $C_2$-$C_6$ alkenyl, halo $C_2$-$C_6$ alkynyl, $C_2$-$C_6$ alkenyloxy, $C_2$-$C_6$ alkynyloxy, halo $C_2$-$C_6$ alkenyloxy, halo $C_2$-$C_6$ alkynyloxy, $C_1$-$C_{10}$ alkylcarbonyloxy, halo $C_1$-$C_{10}$ alkylcarbonyloxy, $C_1$-$C_{10}$ cyanoalkyl, $C_1$-$C_{10}$ cyanoalkoxy, $C_1$-$C_{10}$ alkyl substituted silyl, substituted or unsubstituted amino, aryl, aryl $C_1$-$C_6$ alkyl, aryloxy, aryl $C_1$-$C_6$ alkoxy, arylsulfonyl, arylsulfinyl, arylthio, aryl $C_1$-$C_6$ alkylsulfonyl, aryl $C_1$-$C_6$ alkylsulfinyl, aryl $C_1$-$C_6$ alkylthio, heterocyclyl, heterocyclyl $C_1$-$C_6$ alkyl, and heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3- to 8-membered ring;

$Z^1$, $Z^2$, $Z^3$, and $Z^4$ are each independently selected from H, halogen, CN, $NO_2$, $C_1$-$C_{10}$ alkyl, halo $C_1$-$C_{10}$ alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy, halo $C_1$-$C_{10}$ alkoxy, $C_1$-$C_{10}$ alkylthio, halo $C_1$-$C_{10}$ alkylthio, $C_1$-$C_{10}$ alkylsulfinyl, halo $C_1$-$C_{10}$ alkylsulfinyl, $C_1$-$C_{10}$ alkylsulfonyl, halo $C_1$-$C_{10}$ alkylsulfonyl, formyl, $C_1$-$C_{10}$ alkylcarbonyl, halo $C_1$-$C_{10}$ alkylcarbonyl, $C_1$-$C_{10}$ alkoxycarbonyl, halo $C_1$-$C_{10}$ alkoxycarbonyl, arylcarbonyl, aryloxycarbonyl, $C_2$-$C_6$ alkenyl, $C_2$-$C_6$ alkynyl, halo $C_2$-$C_6$ alkenyl, halo $C_2$-$C_6$ alkynyl, $C_2$-$C_6$ alkenyloxy, $C_2$-$C_6$ alkynyloxy, halo $C_2$-$C_6$ alkenyloxy, halo $C_2$-$C_6$ alkynyloxy, $C_1$-$C_{10}$ alkylcarbonyloxy, halo $C_1$-$C_{10}$ alkylcarbonyloxy, $C_1$-$C_{10}$ cyanoalkyl, $C_1$-$C_{10}$ cyanoalkoxy, $C_1$-$C_{10}$ alkyl substituted silyl, substituted or unsubstituted amino, aryl, aryl $C_1$-$C_6$ alkyl, aryloxy, aryl $C_1$-$C_6$ alkoxy, arylsulfonyl, arylsulfinyl, arylthio, aryl $C_1$-$C_6$ alkylsulfonyl, aryl $C_1$-$C_6$ alkylsulfinyl, aryl $C_1$-$C_6$ alkylthio, heterocyclyl, heterocyclyl $C_1$-$C_6$ alkyl, and heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine;

preferably, in the formula (I),

R is selected from $C_2$-$C_8$ alkyl, halo $C_2$-$C_8$ alkyl, $C_3$-$C_8$ cycloalkyl substituted $C_2$-$C_8$ alkyl, halo $C_3$-$C_8$ cycloalkyl substituted $C_2$-$C_8$ alkyl, $C_3$-$C_8$ cycloalkyl substituted halo $C_2$-$C_8$ alkyl, $C_1$-$C_8$ alkoxy substituted $C_2$-$C_8$ alkyl, halo $C_1$-$C_8$ alkoxy substituted $C_2$-$C_8$ alkyl, $C_1$-$C_8$ alkoxy substituted halo $C_2$-$C_8$ alkyl, $C_3$-$C_8$ cycloalkoxy substituted $C_2$-$C_8$ alkyl, halo $C_3$-$C_8$ cycloalkoxy substituted $C_2$-$C_8$ alkyl, $C_3$-$C_8$ cycloalkoxy substituted halo $C_2$-$C_8$ alkyl, $C_3$-$C_8$ cycloalkyl, halo $C_3$-$C_8$ cycloalkyl, $C_1$-$C_8$ alkyl substituted $C_3$-$C_8$ cycloalkyl, halo $C_1$-$C_8$ alkyl substituted $C_3$-$C_8$ cycloalkyl, $C_1$-$C_8$ alkyl substituted halo $C_3$-$C_8$ cycloalkyl, $C_1$-$C_8$ alkoxy substituted $C_3$-$C_8$ cycloalkyl, halo $C_1$-$C_8$ alkoxy substituted $C_3$-$C_8$ cycloalkyl, $C_1$-$C_8$ alkoxy substituted halo $C_3$-$C_8$ cycloalkyl, $C_3$-$C_8$ cycloalkoxy substituted $C_3$-$C_8$ cycloalkyl, halo $C_3$-$C_8$ cycloalkoxy substituted $C_3$-$C_8$ cycloalkyl, $C_3$-$C_8$ cycloalkoxy substituted halo $C_3$-$C_8$ cycloalkyl, $C_2$-$C_8$ alkenyl, halo $C_2$-$C_8$ alkenyl, $C_2$-$C_8$ alkynyl, and halo $C_2$-$C_8$ alkynyl;

$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_3$-$C_8$ cycloalkyl substituted $C_1$-$C_8$ alkyl, halo $C_3$-$C_8$ cycloalkyl substituted $C_1$-$C_8$ alkyl, $C_3$-$C_8$ cycloalkyl substituted halo $C_1$-$C_8$ alkyl, $C_1$-$C_8$ alkoxy substituted $C_1$-$C_8$ alkyl, halo $C_1$-$C_8$ alkoxy substituted $C_1$-$C_8$ alkyl, $C_1$-$C_8$ alkoxy substituted halo $C_1$-$C_8$ alkyl, $C_3$-$C_8$ cycloalkyl, halo $C_3$-$C_8$ cycloalkyl, $C_1$-$C_8$ alkyl substituted $C_3$-$C_8$ cycloalkyl, halo $C_1$-$C_8$ alkyl substituted $C_3$-$C_8$ cycloalkyl, $C_1$-$C_8$ alkyl substituted halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl;

$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_3$-$C_8$ cycloalkyl, halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl; and

when $Z^1$, $Z^2$, $Z^3$, $Z^4$, $Y^1$, $Y^2$, $Y^3$, $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

**3.** The compound or the salt thereof according to claim 1 or 2, wherein in the formula (I),

R is selected from $C_2$-$C_8$ alkyl, halo $C_2$-$C_8$ alkyl, $C_3$-$C_8$ cycloalkyl, halo $C_3$-$C_8$ cycloalkyl, $C_2$-$C_8$ alkenyl, halo $C_2$-$C_8$ alkenyl, $C_2$-$C_8$ alkynyl and halo $C_2$-$C_8$ alkynyl;
$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_3$-$C_8$ cycloalkyl, halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl;
$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, halo $C_1$-$C_8$ alkylsulfonyl; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

**4.** The compound or the salt thereof according to any one of claims 1-3, wherein in the formula (I),

R is selected from $C_2$-$C_8$ alkyl, halo $C_2$-$C_8$ alkyl, $C_3$-$C_8$ cycloalkyl, halo $C_3$-$C_8$ cycloalkyl, $C_2$-$C_5$ alkenyl, and $C_2$-$C_5$ alkynyl;
$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_1$-$C_8$ alkoxy, and halo $C_1$-$C_8$ alkoxy;
$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_1$-$C_8$ alkoxy, and halo $C_1$-$C_8$ alkoxy; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine;
preferably, in the formula (I),
R is selected from ethyl, n-propyl, isopropyl, cyclopropyl, n-butyl, sec-butyl, isobutyl, tert-butyl, allyl, propargyl, and $CF_3$;
$Y^1$, $Y^2$, $Y^3$, $Y^4$, and $Y^5$ are each independently selected from H, F, Cl, Br, I, CN, $NO_2$, $CH_3$, $CF_3$, $OCH_3$, $OCF_3$, $OCH_2CF_3$, $OCH_2CF_2CF_3$, $CF_2Cl$, $CFCl_2$, and $CCl_3$;
$Z^1$, $Z^2$, $Z^3$, and $Z^4$ are each independently selected from H, F, Cl, Br, I, CN, $NO_2$, $CH_3$, $CF_3$, $OCF_3$, ethyl, n-propyl, isopropyl, n-butyl, sec-butyl, isobutyl, and tert-butyl; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine;
more preferably, in the formula (I),
R is selected from ethyl, n-propyl, isopropyl, n-butyl, sec-butyl, and isobutyl;
$Y^1$, $Y^2$, $Y^3$, $Y^4$, and $Y^5$ are each independently selected from H, F, Cl, Br, CN, $NO_2$, $OCF_3$, $CH_3$, $CF_3$, $OCH_3$, $OCH_2CF_3$, and $OCH_2CF_2CF_3$;
$Z^1$, $Z^2$, $Z^3$, and $Z^4$ are each independently selected from H, F, Cl, Br, and $CH_3$; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

**5.** A method for preparing a benzimidazole compound, comprising:

(1) subjecting a compound V and a compound IV to a first reaction in a first solvent in the presence of a first alkaline substance and a condensing agent to obtain a compound III;
(2) subjecting the compound III and an acidic substance to a second reaction in a second solvent to obtain a compound II; and
(3) subjecting the compound II and a sulfonyl-containing compound to a third reaction in a third solvent in the presence of a second alkaline substance to obtain the benzimidazole compound;

wherein the compound V has a structure represented by a formula (V), the compound IV has a structure represented

by a formula (IV), the compound III has a structure represented by a formula (III), the compound II has a structure represented by a formula (II), the benzimidazole compound has a structure represented by a formula (I), and the sulfonyl-containing compound has a structure represented by a formula (VI);

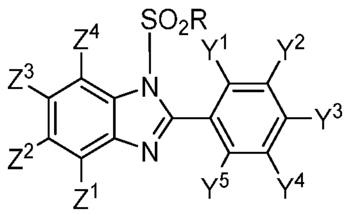 Formula (I); 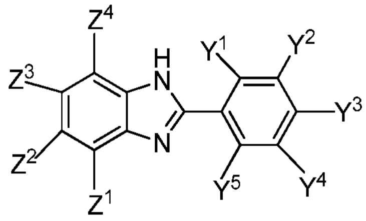 Formula (II);

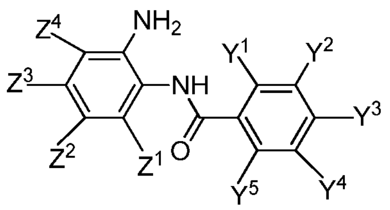 Formula (III); 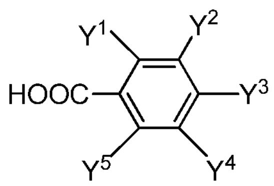 Formula (IV);

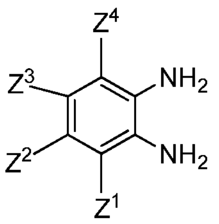 Formula (V); 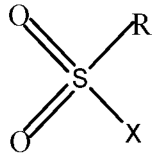 Formula (VI);

in the formula (I), the formula (II), the formula (III), the formula (IV), the formula (V) and the formula (VI), definitions of R, $Y^1$, $Y^2$, $Y^3$, $Y^4$, $Y^5$, $Z^1$, $Z^2$, $Z^3$, and $Z^4$ are correspondingly the same as those in any one of claims 1-4, and X is selected from halogen.

**6.** The method according to claim 5, wherein in the step (1), the conditions of the first reaction comprises: a temperature of -10°C to 150°C, and a reaction time of 0.5-48 h;

preferably, the compound V and the compound IV are used in a molar ratio of 0.5-2:1;
preferably, the condensing agent is selected from at least one of 1-ethyl-3-(3-dimethylaminopropyl)carbodiimide or a hydrochloride thereof, carbonyldiimidazole, 1,3-dicyclohexylcarbodiimide, diethyl cyanophosphate, chlorocarbonates, and 2-chloro-1-methylpyridinium iodide;
preferably, the condensing agent and the compound IV are used in a molar ratio of 1-2:1;
preferably, the first alkaline substance is selected from at least one of pyridine, dimethylaminopyridine, triethylamine, diisopropylethylamine, sodium hydroxide, potassium hydroxide, sodium carbonate, potassium carbonate, sodium bicarbonate, potassium bicarbonate, sodium acetate, potassium acetate, sodium methoxide, sodium ethoxide, sodium tert-butoxide, potassium tert-butoxide, and 1,8-diazabicyclo[5.4.0]undec-7-ene; and
preferably, the first alkaline substance and the compound IV are used in a molar ratio of 0.1-10:1.

**7.** The method according to claim 5 or 6, wherein in the step (2), the conditions of the second reaction include: a temperature of -10°C to 300°C, and a reaction time of 0.5-48 h;

preferably, the acidic substance is selected from at least one of p-toluenesulfonic acid or a hydrate thereof, methanesulfonic acid, trifluoromethanesulfonic acid, hydrochloric acid, sulfuric acid, nitric acid, formic acid, acetic acid, propionic acid, trifluoroacetic acid, trichloroacetic acid, benzoic acid, and phosphoric acid; and
preferably, the acidic substance and the compound IV are used in a molar ratio of 0.01-10:1.

**8.** The method according to any one of claims 5-7, wherein in the step (3), the conditions of the third reaction comprise:

a temperature of -10°C to 100°C, and a reaction time of 0.5-48 h;

preferably, the sulfonyl-containing compound and the compound II are used in a molar ratio of 0.8-10:1; preferably, the second alkaline substance is selected from at least one of pyridine, dimethylaminopyridine, triethylamine, diisopropylethylamine, sodium hydride, potassium hydride, sodium hydroxide, potassium hydroxide, sodium carbonate, potassium carbonate, sodium bicarbonate, potassium bicarbonate, sodium acetate, potassium acetate, sodium methoxide, sodium ethoxide, sodium tert-butoxide, potassium tert-butoxide, and 1,8-diazabicyclo[5.4.0]undec-7-ene; and
preferably, the second alkaline substance and the compound II are used in a molar ratio of 1-10:1.

**9.** A benzimidazole compound prepared by the method according to any one of claims 5-8.

**10.** Use of the benzimidazole compound or the salt thereof according to any one of claims 1 to 4 and 9 for the preparation of an insecticide and acaricide.

**11.** An insecticide and acaricide, comprising an active ingredient selected from at least one of the benzimidazole compound or the salt thereof according to any one of claims 1 to 4 and 9; wherein
preferably, the content of the active ingredient ranges from 1 wt% to 99 wt%, more preferably from 5 wt% to 60 wt% based on the total weight of the insecticide and acaricide.

**12.** Use of the insecticide and acaricide according to claim 11 for killing pests and/or mites in agriculture, forestry and horticulture.

**Amended claims under Art. 19.1 PCT**

**1.** A benzimidazole compound or a salt thereof, wherein the benzimidazole compound has a structure represented by a formula (I):

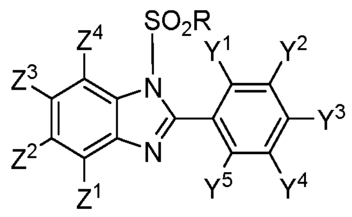
Formula (I);

wherein in the formula (I),

R is selected from substituted or unsubstituted $C_2$-$C_{10}$ alkyl, substituted or unsubstituted $C_3$-$C_{10}$ cycloalkyl, substituted or unsubstituted $C_2$-$C_{10}$ alkenyl, and substituted or unsubstituted $C_2$-$C_{10}$ alkynyl, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy;
$Y^1$, $Y^2$, $Y^3$, $Y^4$, and $Y^5$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted

heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3- to 8-membered ring;

$Z^1$, $Z^2$, $Z^3$, and $Z^4$ are each independently selected from H, halogen, CN, $NO_2$, formyl, substituted or unsubstituted $C_1$-$C_{10}$ alkyl, substituted or unsubstituted $C_3$-$C_{10}$ cycloalkyl, substituted or unsubstituted $C_1$-$C_{10}$ alkoxy, substituted or unsubstituted $C_1$-$C_{10}$ alkylthio, substituted or unsubstituted $C_1$-$C_{10}$ alkylsulfinyl, substituted or unsubstituted $C_1$-$C_{10}$ alkylsulfonyl, substituted or unsubstituted $C_1$-$C_{10}$ alkylcarbonyl, substituted or unsubstituted $C_1$-$C_{10}$ alkoxycarbonyl, substituted or unsubstituted arylcarbonyl, substituted or unsubstituted aryloxycarbonyl, substituted or unsubstituted $C_2$-$C_6$ alkenyl, substituted or unsubstituted $C_2$-$C_6$ alkynyl, substituted or unsubstituted $C_2$-$C_6$ alkenyloxy, substituted or unsubstituted $C_2$-$C_6$ alkynyloxy, substituted or unsubstituted $C_1$-$C_{10}$ alkylcarbonyloxy, substituted or unsubstituted $C_1$-$C_{10}$ cyanoalkyl, substituted or unsubstituted $C_1$-$C_{10}$ cyanoalkoxy, substituted or unsubstituted silyl, substituted or unsubstituted amino, substituted or unsubstituted aryl, substituted or unsubstituted aryl $C_1$-$C_6$ alkyl, substituted or unsubstituted aryloxy, substituted or unsubstituted aryl $C_1$-$C_6$ alkoxy, substituted or unsubstituted arylsulfonyl, substituted or unsubstituted arylsulfinyl, substituted or unsubstituted arylthio, substituted or unsubstituted aryl $C_1$-$C_6$ alkylsulfonyl, substituted or unsubstituted aryl $C_1$-$C_6$ alkylsulfinyl, substituted or unsubstituted aryl $C_1$-$C_6$ alkylthio, substituted or unsubstituted heterocyclyl, substituted or unsubstituted heterocyclyl $C_1$-$C_6$ alkyl, and substituted or unsubstituted heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

**2.** The compound or the salt thereof according to claim 1, wherein in the formula (I),

R is selected from $C_2$-$C_{10}$ alkyl, halo $C_2$-$C_{10}$ alkyl, $C_3$-$C_{10}$ cycloalkyl substituted $C_2$-$C_{10}$ alkyl, halo $C_3$-$C_{10}$ cycloalkyl substituted $C_2$-$C_{10}$ alkyl, $C_3$-$C_{10}$ cycloalkyl substituted halo $C_2$-$C_{10}$ alkyl, $C_1$-$C_{10}$ alkoxy substituted $C_2$-$C_{10}$ alkyl, halo $C_1$-$C_{10}$ alkoxy substituted $C_2$-$C_{10}$ alkyl, $C_1$-$C_{10}$ alkoxy substituted halo $C_2$-$C_{10}$ alkyl, $C_3$-$C_{10}$ cycloalkoxy substituted $C_2$-$C_{10}$ alkyl, halo $C_3$-$C_{10}$ cycloalkoxy substituted $C_2$-$C_{10}$ alkyl, $C_3$-$C_{10}$ cycloalkoxy substituted halo $C_2$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy substituted $C_3$-$C_{10}$ cycloalkyl, halo $C_1$-$C_{10}$ alkoxy substituted $C_3$-$C_{10}$ cycloalkyl, $C_1$-$C_{10}$ alkoxy substituted halo $C_3$-$C_{10}$ cycloalkyl, $C_3$-$C_{10}$ cycloalkoxy substituted $C_3$-$C_{10}$ cycloalkyl, halo $C_3$-$C_{10}$ cycloalkoxy substituted $C_3$-$C_{10}$ cycloalkyl, $C_3$-$C_{10}$ cycloalkoxy substituted halo $C_3$-$C_{10}$ cycloalkyl, $C_2$-$C_{10}$ alkenyl, halo $C_2$-$C_{10}$ alkenyl, $C_2$-$C_{10}$ alkynyl, and halo $C_2$-$C_{10}$ alkynyl;

$Y^1$, $Y^2$, $Y^3$, $Y^4$, and $Y^5$ are each independently selected from H, halogen, CN, $NO_2$, $C_1$-$C_{10}$ alkyl, halo $C_1$-$C_{10}$ alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy, halo $C_1$-$C_{10}$ alkoxy, $C_1$-$C_{10}$ alkylthio, halo $C_1$-$C_{10}$ alkylthio, $C_1$-$C_{10}$ alkylsulfinyl, halo $C_1$-$C_{10}$ alkylsulfinyl, $C_1$-$C_{10}$ alkylsulfonyl, halo $C_1$-$C_{10}$ alkylsulfonyl, formyl, $C_1$-$C_{10}$ alkylcarbonyl, halo $C_1$-$C_{10}$ alkylcarbonyl, $C_1$-$C_{10}$ alkoxycarbonyl, halo $C_1$-$C_{10}$ alkoxycarbonyl, arylcarbonyl, aryloxycarbonyl, $C_2$-$C_6$ alkenyl, $C_2$-$C_6$ alkynyl, halo $C_2$-$C_6$ alkenyl, halo $C_2$-$C_6$ alkynyl, $C_2$-$C_6$ alkenyloxy, $C_2$-$C_6$ alkynyloxy, halo $C_2$-$C_6$ alkenyloxy, halo $C_2$-$C_6$ alkynyloxy, $C_1$-$C_{10}$ alkylcarbonyloxy, halo $C_1$-$C_{10}$ alkylcarbonyloxy, $C_1$-$C_{10}$ cyanoalkyl, $C_1$-$C_{10}$ cyanoalkoxy, $C_1$-$C_{10}$ alkyl substituted silyl, substituted or unsubstituted amino, aryl, aryl $C_1$-$C_6$ alkyl, aryloxy, aryl $C_1$-$C_6$ alkoxy, arylsulfonyl, arylsulfinyl, arylthio, aryl $C_1$-$C_6$ alkylsulfonyl, aryl $C_1$-$C_6$ alkylsulfinyl, aryl $C_1$-$C_6$ alkylthio, heterocyclyl, heterocyclyl $C_1$-$C_6$ alkyl, and heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Y^1$, $Y^2$, $Y^3$, $Y^4$, and $Y^5$ form one group, and at least one group and a bonded benzene ring are cyclized with or without at least one heteroatom to form at least one 3-

to 8-membered ring;

$Z^1$, $Z^2$, $Z^3$, and $Z^4$ are each independently selected from H, halogen, CN, $NO_2$, $C_1$-$C_{10}$ alkyl, halo $C_1$-$C_{10}$ alkyl, $C_3$-$C_{10}$ cycloalkyl substituted $C_1$-$C_{10}$ alkyl, halo $C_3$-$C_{10}$ cycloalkyl substituted $C_1$-$C_{10}$ alkyl, $C_3$-$C_{10}$ cycloalkyl substituted halo $C_1$-$C_{10}$ alkyl, $C_1$-$C_{10}$ alkoxy substituted $C_1$-$C_{10}$ alkyl, halo $C_1$-$C_{10}$ alkoxy substituted $C_1$-$C_{10}$ alkyl, $C_1$-$C_{10}$ alkoxy substituted halo $C_1$-$C_{10}$ alkyl, $C_3$-$C_{10}$ cycloalkyl, halo $C_3$-$C_{10}$ cycloalkyl, $C_1$-$C_{10}$ alkyl substituted $C_3$-$C_{10}$ cycloalkyl, halo $C_1$-$C_{10}$ alkyl substituted $C_3$-$C_{10}$ cycloalkyl, $C_1$-$C_{10}$ alkyl substituted halo $C_3$-$C_{10}$ cycloalkyl, $C_1$-$C_{10}$ alkoxy, halo $C_1$-$C_{10}$ alkoxy, $C_1$-$C_{10}$ alkylthio, halo $C_1$-$C_{10}$ alkylthio, $C_1$-$C_{10}$ alkylsulfinyl, halo $C_1$-$C_{10}$ alkylsulfinyl, $C_1$-$C_{10}$ alkylsulfonyl, halo $C_1$-$C_{10}$ alkylsulfonyl, formyl, $C_1$-$C_{10}$ alkylcarbonyl, halo $C_1$-$C_{10}$ alkylcarbonyl, $C_1$-$C_{10}$ alkoxycarbonyl, halo $C_1$-$C_{10}$ alkoxycarbonyl, arylcarbonyl, aryloxycarbonyl, $C_2$-$C_6$ alkenyl, $C_2$-$C_6$ alkynyl, halo $C_2$-$C_6$ alkenyl, halo $C_2$-$C_6$ alkynyl, $C_2$-$C_6$ alkenyloxy, $C_2$-$C_6$ alkynyloxy, halo $C_2$-$C_6$ alkenyloxy, halo $C_2$-$C_6$ alkynyloxy, $C_1$-$C_{10}$ alkylcarbonyloxy, halo $C_1$-$C_{10}$ alkylcarbonyloxy, $C_1$-$C_{10}$ cyanoalkyl, $C_1$-$C_{10}$ cyanoalkoxy, $C_1$-$C_{10}$ alkyl substituted silyl, substituted or unsubstituted amino, aryl, aryl $C_1$-$C_6$ alkyl, aryloxy, aryl $C_1$-$C_6$ alkoxy, arylsulfonyl, arylsulfinyl, arylthio, aryl $C_1$-$C_6$ alkylsulfonyl, aryl $C_1$-$C_6$ alkylsulfinyl, aryl $C_1$-$C_6$ alkylthio, heterocyclyl, heterocyclyl $C_1$-$C_6$ alkyl, and heterocyclyloxy, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy; and optionally, any two adjacent groups of $Z^1$, $Z^2$, $Z^3$, and $Z^4$ form one group, and at least one group and a bonded benzene ring are cyclized with or without at least one heteroatom to form at least one 3- to 8-membered ring; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine;

preferably, in the formula (I),

R is selected from $C_2$-$C_8$ alkyl, halo $C_2$-$C_8$ alkyl, $C_3$-$C_8$ cycloalkyl substituted $C_2$-$C_8$ alkyl, halo $C_3$-$C_8$ cycloalkyl substituted $C_2$-$C_8$ alkyl, $C_3$-$C_8$ cycloalkyl substituted halo $C_2$-$C_8$ alkyl, $C_1$-$C_8$ alkoxy substituted $C_2$-$C_8$ alkyl, halo $C_1$-$C_8$ alkoxy substituted $C_2$-$C_8$ alkyl, $C_1$-$C_8$ alkoxy substituted halo $C_2$-$C_8$ alkyl, $C_3$-$C_8$ cycloalkoxy substituted $C_2$-$C_8$ alkyl, halo $C_3$-$C_8$ cycloalkoxy substituted $C_2$-$C_8$ alkyl, $C_3$-$C_8$ cycloalkoxy substituted halo $C_2$-$C_8$ alkyl, $C_3$-$C_8$ cycloalkyl, halo $C_3$-$C_8$ cycloalkyl, $C_1$-$C_8$ alkyl substituted $C_3$-$C_8$ cycloalkyl, halo $C_1$-$C_8$ alkyl substituted $C_3$-$C_8$ cycloalkyl, $C_1$-$C_8$ alkyl substituted halo $C_3$-$C_8$ cycloalkyl, $C_1$-$C_8$ alkoxy substituted $C_3$-$C_8$ cycloalkyl, halo $C_1$-$C_8$ alkoxy substituted $C_3$-$C_8$ cycloalkyl, $C_1$-$C_8$ alkoxy substituted halo $C_3$-$C_8$ cycloalkyl, $C_3$-$C_8$ cycloalkoxy substituted $C_3$-$C_8$ cycloalkyl, halo $C_3$-$C_8$ cycloalkoxy substituted $C_3$-$C_8$ cycloalkyl, $C_3$-$C_8$ cycloalkoxy substituted halo $C_3$-$C_8$ cycloalkyl, $C_2$-$C_8$ alkenyl, halo $C_2$-$C_8$ alkenyl, $C_2$-$C_8$ alkynyl, and halo $C_2$-$C_8$ alkynyl;

$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_3$-$C_8$ cycloalkyl substituted $C_1$-$C_8$ alkyl, halo $C_3$-$C_8$ cycloalkyl substituted $C_1$-$C_8$ alkyl, $C_3$-$C_8$ cycloalkyl substituted halo $C_1$-$C_8$ alkyl, $C_1$-$C_8$ alkoxy substituted $C_1$-$C_8$ alkyl, halo $C_1$-$C_8$ alkoxy substituted $C_1$-$C_8$ alkyl, $C_1$-$C_8$ alkoxy substituted halo $C_1$-$C_8$ alkyl, $C_3$-$C_8$ cycloalkyl, halo $C_3$-$C_8$ cycloalkyl, $C_1$-$C_8$ alkyl substituted $C_3$-$C_8$ cycloalkyl, halo $C_1$-$C_8$ alkyl substituted $C_3$-$C_8$ cycloalkyl, $C_1$-$C_8$ alkyl substituted halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl;

$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_3$-$C_8$ cycloalkyl, halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl; and

when $Z^1$, $Z^2$, $Z^3$, $Z^4$, $Y^1$, $Y^2$, $Y^3$, $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

**3.** The compound or the salt thereof according to claim 1 or 2, wherein in the formula (I),

R is selected from $C_2$-$C_8$ alkyl, halo $C_2$-$C_8$ alkyl, $C_3$-$C_8$ cycloalkyl, halo $C_3$-$C_8$ cycloalkyl, $C_2$-$C_8$ alkenyl, halo $C_2$-$C_8$ alkenyl, $C_2$-$C_8$ alkynyl and halo $C_2$-$C_8$ alkynyl;

$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_3$-$C_8$ cycloalkyl, halo $C_3$-$C_8$ cycloalkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, and halo $C_1$-$C_8$ alkylsulfonyl;

$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_1$-$C_8$ alkoxy, halo $C_1$-$C_8$ alkoxy, $C_1$-$C_8$ alkylthio, halo $C_1$-$C_8$ alkylthio, $C_1$-$C_8$ alkylsulfinyl, halo $C_1$-$C_8$ alkylsulfinyl, $C_1$-$C_8$ alkylsulfonyl, halo $C_1$-$C_8$ alkylsulfonyl; and

when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$,

and $Y^5$ are all H and R is ethyl, $Y^2$ and $Y^4$ are not simultaneously chlorine.

4. The compound or the salt thereof according to any one of claims 1-3, wherein in the formula (I),

R is selected from $C_2$-$C_8$ alkyl, halo $C_2$-$C_8$ alkyl, $C_3$-$C_8$ cycloalkyl, halo $C_3$-$C_8$ cycloalkyl, $C_2$-$C_5$ alkenyl, and $C_2$-$C_5$ alkynyl;
$Y^1$, $Y^2$, $Y^3$, $Y^4$, and $Y^5$ are each independently selected from H, halogen, CN, $NO_2$, $C_1$-$C_8$ alkyl, halo $C_1$-$C_8$ alkyl, $C_1$-$C_8$ alkoxy, and halo $C_1$-$C_8$ alkoxy;
$Z^1$, $Z^2$, $Z^3$, and $Z^4$ are each independently selected from H, halogen, CN, $NO_2$, $C_1$-$C_8$ alkyl, halo $C_1$-$C_8$ alkyl, $C_1$-$C_8$ alkoxy, and halo $C_1$-$C_8$ alkoxy; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine;
preferably, in the formula (I),
R is selected from ethyl, n-propyl, isopropyl, cyclopropyl, n-butyl, sec-butyl, isobutyl, tert-butyl, allyl, propargyl, and $CF_3$;
$Y^1$, $Y^2$, $Y^3$, $Y^4$, and $Y^5$ are each independently selected from H, F, Cl, Br, I, CN, $NO_2$, $CH_3$, $CF_3$, $OCH_3$, $OCF_3$, $OCH_2CF_3$, $OCH_2CF_2CF_3$, $CF_2Cl$, $CFCl_2$, and $CCl_3$;
$Z^1$, $Z^2$, $Z^3$, and $Z^4$ are each independently selected from H, F, Cl, Br, I, CN, $NO_2$, $CH_3$, $CF_3$, $OCF_3$, ethyl, n-propyl, isopropyl, n-butyl, sec-butyl, isobutyl, and tert-butyl; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine;
more preferably, in the formula (I),
R is selected from ethyl, n-propyl, isopropyl, n-butyl, sec-butyl, and isobutyl;
$Y^1$, $Y^2$, $Y^3$, $Y^4$, and $Y^5$ are each independently selected from H, F, Cl, Br, CN, $NO_2$, $OCF_3$, $CH_3$, $CF_3$, $OCH_3$, $OCH_2CF_3$, and $OCH_2CF_2CF_3$;
$Z^1$, $Z^2$, $Z^3$, and $Z^4$ are each independently selected from H, F, Cl, Br, and $CH_3$; and
when $Z^1$, $Z^2$, $Z^3$, $Z^4$, $Y^1$, $Y^2$, $Y^3$, and $Y^4$ are all H and R is ethyl, $Y^5$ is not H, fluorine or chlorine; when $Z^1$, $Z^2$, $Z^3$, $Z^4$, $Y^1$, $Y^2$, $Y^4$, and $Y^5$ are all H and R is ethyl, $Y^3$ is not fluorine or chlorine; and when $Z^1$, $Z^2$, $Z^3$, $Z^4$, $Y^1$, $Y^3$, and $Y^5$ are all H and R is ethyl, $Y^2$ and $Y^4$ are not simultaneously chlorine.

5. A method for preparing a benzimidazole compound, comprising:

(1) subjecting a compound V and a compound IV to a first reaction in a first solvent in the presence of a first alkaline substance and a condensing agent to obtain a compound III;
(2) subjecting the compound III and an acidic substance to a second reaction in a second solvent to obtain a compound II; and
(3) subjecting the compound II and a sulfonyl-containing compound to a third reaction in a third solvent in the presence of a second alkaline substance to obtain the benzimidazole compound;

wherein the compound V has a structure represented by a formula (V), the compound IV has a structure represented by a formula (IV), the compound III has a structure represented by a formula (III), the compound II has a structure represented by a formula (II), the benzimidazole compound has a structure represented by a formula (I), and the sulfonyl-containing compound has a structure represented by a formula (VI);

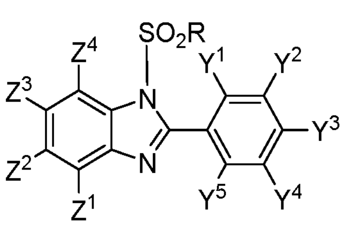 Formula (I); 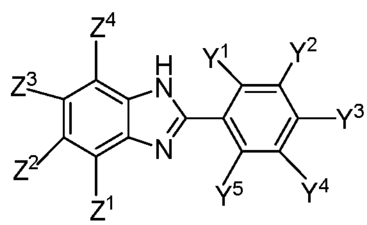 Formula (II);

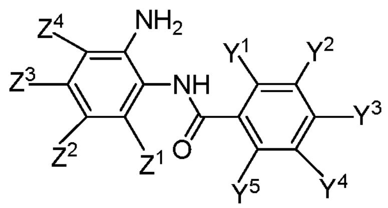 Formula (III); 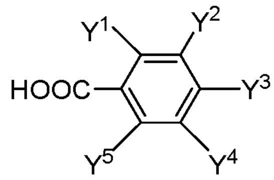 Formula (IV);

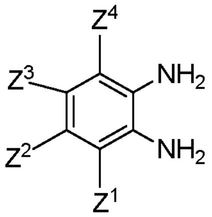 Formula (V); 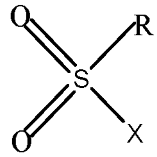 Formula (VI);

in the formula (I), the formula (II), the formula (III), the formula (IV), the formula (V) and the formula (VI), definitions of R, $Y^1$, $Y^2$, $Y^3$, $Y^4$, $Y^5$, $Z^1$, $Z^2$, $Z^3$, and $Z^4$ are correspondingly the same as those in any one of claims 1-4, and X is selected from halogen.

**6.** The method according to claim 5, wherein in the step (1), the conditions of the first reaction comprises: a temperature of -10°C to 150°C, and a reaction time of 0.5-48 h;

preferably, the compound V and the compound IV are used in a molar ratio of 0.5-2:1;
preferably, the condensing agent is selected from at least one of 1-ethyl-3-(3-dimethylaminopropyl)carbodiimide or a hydrochloride thereof, carbonyldiimidazole, 1,3-dicyclohexylcarbodiimide, diethyl cyanophosphate, chlorocarbonates, and 2-chloro-1-methylpyridinium iodide;
preferably, the condensing agent and the compound IV are used in a molar ratio of 1-2: 1;
preferably, the first alkaline substance is selected from at least one of pyridine, dimethylaminopyridine, triethylamine, diisopropylethylamine, sodium hydroxide, potassium hydroxide, sodium carbonate, potassium carbonate, sodium bicarbonate, potassium bicarbonate, sodium acetate, potassium acetate, sodium methoxide, sodium ethoxide, sodium tert-butoxide, potassium tert-butoxide, and 1,8-diazabicyclo[5.4.0]undec-7-ene; and
preferably, the first alkaline substance and the compound IV are used in a molar ratio of 0.1-10:1.

**7.** The method according to claim 5 or 6, wherein in the step (2), the conditions of the second reaction include: a temperature of -10°C to 300°C, and a reaction time of 0.5-48 h;

preferably, the acidic substance is selected from at least one of p-toluenesulfonic acid or a hydrate thereof, methanesulfonic acid, trifluoromethanesulfonic acid, hydrochloric acid, sulfuric acid, nitric acid, formic acid, acetic acid, propionic acid, trifluoroacetic acid, trichloroacetic acid, benzoic acid, and phosphoric acid; and
preferably, the acidic substance and the compound IV are used in a molar ratio of 0.01-10:1.

**8.** The method according to any one of claims 5-7, wherein in the step (3), the conditions of the third reaction comprise: a temperature of -10°C to 100°C, and a reaction time of 0.5-48 h;

preferably, the sulfonyl-containing compound and the compound II are used in a molar ratio of 0.8-10:1;
preferably, the second alkaline substance is selected from at least one of pyridine, dimethylaminopyridine, triethylamine, diisopropylethylamine, sodium hydride, potassium hydride, sodium hydroxide, potassium hydroxide, sodium carbonate, potassium carbonate, sodium bicarbonate, potassium bicarbonate, sodium acetate, potassium acetate, sodium methoxide, sodium ethoxide, sodium tert-butoxide, potassium tert-butoxide, and 1,8-diazabicyclo[5.4.0]undec-7-ene; and
preferably, the second alkaline substance and the compound II are used in a molar ratio of 1-10: 1.

**9.** A benzimidazole compound prepared by the method according to any one of claims 5-8.

**10.** Use of the benzimidazole compound or the salt thereof according to any one of claims 1 to 4 and 9 for the preparation

of an insecticide and acaricide.

**11.** An insecticide and acaricide, comprising an active ingredient selected from at least one of the benzimidazole compound or the salt thereof according to any one of claims 1 to 4 and 9; wherein
preferably, the content of the active ingredient ranges from 1 wt% to 99 wt%, more preferably from 5 wt% to 60 wt% based on the total weight of the insecticide and acaricide.

**12.** Use of the insecticide and acaricide according to claim 11 for killing pests and/or mites in agriculture, forestry and horticulture.

**Statement under Art. 19.1 PCT**

Amend the originally filed claims in accordance with Article 19 as follows.

Delete the technical solutions in claims 1-4 where R is methyl or substituted methyl to form new claims 1-4.

On novelty

New claim 1, in formula (1), R is selected from substituted or unsubstituted $C_2$-$C_{10}$ alkyl, substituted or unsubstituted $C_3$-$C_{10}$ cycloalkyl, substituted or unsubstituted $C_2$-$C_{10}$ alkenyl, and substituted or unsubstituted $C_2$-$C_{10}$ alkynyl, and optionally present substituents are each independently selected from at least one of halogen, $C_1$-$C_{10}$ alkyl, halo $C_1$-$C_{10}$ alkyl, $C_3$-$C_{10}$ cycloalkyl, halo $C_3$-$C_{10}$ cycloalkyl, $C_1$-$C_{10}$ alkoxy, halo $C_1$-$C_{10}$ alkoxy, $C_3$-$C_{10}$ cycloalkoxy, and halo $C_3$-$C_{10}$ cycloalkoxy, i.e., R is not methyl and substituted methyl.

Whereas D1 discloses compounds in which the substituent at the position corresponding to R is methyl. Therefore, the new claim 1 has a different substituent at the position of R as compared to D1. Based on the aforesaid distinguishing technical features, the new claim 1 is novel compared with D1 and complies with PCT Article 33(2). Similarly, compared to D2-D6, the new claim 1 is novel compared with D2-D6 respectively due to the different substituents at the R position, and complies with PCT Article 33(2).

On the basis of the novelty of the new claim 1, and for the same reasons stated above, new claims 2-4 and 9 are novel and comply with PCT Article 33(2).

On inventiveness

Based on the aforementioned distinguishing technical features, the present application seeks to solve the technical problem of providing a benzimidazole compound or a salt thereof that can be used as an insecticide or acaricide. In this regard, none of the prior art, including D1-D6, teaches or reveals that benzimidazole compounds have insecticidal and acaricidal activity. Therefore, in solving the above technical problem, the person skilled in the art is not motivated to specifically make substitutions of the R substituent, nor can he anticipate that the compounds obtained by substituting R substituents can have insecticidal and acaricidal activity. Therefore, the new claim 1 is inventive and complies with PCT Article 33(2).

On the basis of the inventiveness of the new claim 1, and for the same reasons stated above, new claims 2-4 and 9 are inventive and comply with PCT Article 33(2).

**INTERNATIONAL SEARCH REPORT**

International application No. **PCT/CN2022/077277**

**A. CLASSIFICATION OF SUBJECT MATTER**

C07D 235/22(2006.01)i; C07D 235/04(2006.01)i; C07D 235/02(2006.01)i; C07D 235/00(2006.01)i; A01N 43/52(2006.01)i; A01P 7/00(2006.01)i

According to International Patent Classification (IPC) or to both national classification and IPC

**B. FIELDS SEARCHED**

Minimum documentation searched (classification system followed by classification symbols)

C07D; A01N; A01P

Documentation searched other than minimum documentation to the extent that such documents are included in the fields searched

Electronic data base consulted during the international search (name of data base and, where practicable, search terms used)

CNTXT; ENTXT; CNPAT; CNKI; STN; Insecticidal, pesticide, insectifuge, Acaricide, Acarid, Acarus, mite, disinsection, 杀虫, 杀螨, 苯并咪唑

**C. DOCUMENTS CONSIDERED TO BE RELEVANT**

| Category* | Citation of document, with indication, where appropriate, of the relevant passages | Relevant to claim No. |
|---|---|---|
| X | CN 111187219 A (MAANSHAN HIGH-TECH RESEARCH INSTITUTE OF NANJING UNIVERSITY CO., LTD.) 22 May 2020 (2020-05-22)<br>embodiment 1, compound B1 | 1-4, 9 |
| X | CN 106946790 A (HUAQIAO UNIVERSITY) 14 July 2017 (2017-07-14)<br>embodiment 19 | 1-4, 9 |
| X | ALAM, M.T., et al. "An Intramolecular C(sp2)–H Amidation Using N-Iodosuccinimide"<br>*Eur. J. Org. Chem.,* Vol. no. 2018, No. 30, 31 December 2018 (2018-12-31),<br>pp. 4178-4186 | 1-4, 9 |
| X | RAUT, C.N., et al. "Microwave-Mediated Synthesis and Antibacterial Activity of Some Novel 2-(Substituted Biphenyl) Benzimidazoles via Suzuki-Miyaura Cross Coupling Reaction and Their N-Substituted Derivatives"<br>*J. Heterocyclic Chem.,* Vol. 48, No. 2, 31 March 2011 (2011-03-31),<br>pp. 419-425 | 1-4, 9 |
| X | REGISTRY. "RN 1629362-60-2"<br>*ACS on STN,* 21 October 2014 (2014-10-21), | 1-4, 9 |
| X | REGISTRY. "RN 1618652-83-7"<br>*ACS on STN,* 31 July 2014 (2014-07-31), | 1-4, 9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 April 2022** | **17 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/077277**

**C. DOCUMENTS CONSIDERED TO BE RELEVANT**

| Category* | Citation of document, with indication, where appropriate, of the relevant passages | Relevant to claim No. |
|---|---|---|
| A | CN 111187219 A (MAANSHAN HIGH-TECH RESEARCH INSTITUTE OF NANJING UNIVERSITY CO., LTD.) 22 May 2020 (2020-05-22)<br>embodiment 1, compound B1 | 5-8, 10-12 |
| A | CN 106946790 A (HUAQIAO UNIVERSITY) 14 July 2017 (2017-07-14)<br>embodiment 19 | 5-8, 10-12 |

Form PCT/ISA/210 (second sheet) (January 2015)

**INTERNATIONAL SEARCH REPORT**
**Information on patent family members**

International application No.
**PCT/CN2022/077277**

| Patent document cited in search report | | | Publication date (day/month/year) | Patent family member(s) | Publication date (day/month/year) |
|---|---|---|---|---|---|
| CN | 111187219 | A | 22 May 2020 | None | |
| CN | 106946790 | A | 14 July 2017 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202110204832

### Non-patent literature cited in the description

- *Advanced Materials Research,* 2011, vol. 236-238, 2570-2573